# EUROPEAN PATENT SPECIFICATION

(11) **EP 3 079 090 B1**
(45) Date of publication and mention of the grant of the patent: **09.03.2022**
(21) Application number: 16164069.3
(22) Date of filing: 06.04.2016
(51) Int. Cl.: G16H 20/30, G16H 40/67

(54) **METHOD FOR PROCESSING DATA AND ELECTRONIC DEVICE THEREOF**
VERFAHREN ZUR VERARBEITUNG VON DATEN UND ELEKTRONISCHE VORRICHTUNG DAFÜR
PROCÉDÉ DE TRAITEMENT DE DONNÉES ET DISPOSITIF ÉLECTRONIQUE ASSOCIÉ

(30) Priority: 06.04.2015 KR 20150048532
(43) Date of publication of application: 12.10.2016
(73) Proprietor: Samsung Electronics Co., Ltd., Gyeonggi-do 16677 (KR)
(72) Inventor: HAN, Jooman, Gyeonggi-do (KR); PARK, Hyung-Jin, Gyeonggi-do (KR); LEE, Kihuk, Gyeonggi-do (KR); HAN, Dongsoo, Seoul (KR); HAM, Dongeup, Gyeonggi-do (KR); KU, Sangchul, Gyeonggi-do (KR); JEON, Yongjoon, Gyeonggi-do (KR)
(74) Representative: HGF

(56) References cited:
- US-A1- 2014 249 853
- US-A1- 2014 278 220
- US-A1- 2014 335 490

## Description

### TECHNICAL FIELD

Various example embodiments of the present disclosure relate to a method for processing data in an electronic device and the electronic device thereof.

### BACKGROUND

An electronic device can perform a set function based on a user input. When the electronic device performs the set function, the electronic device can output a corresponding interface through a display or speaker, and can transmit designated information to another electronic device through a network communication, and can transmit a control signal to control the another electronic device.

The electronic device can perform an operation corresponding to a user input, but many errors occur at a time the electronic device determines a specific situation with reference to biometric information of the user and determines an operation corresponding to the specific situation.

The electronic device can acquire various users' biometric information, and determine an operation corresponding to a specific situation or learn a new situation based on the acquired biometric information.

When the electronic device predicts a behavior of a user correspondingly to information that is measured through a sensor in an unexpected situation, the electronic device is lower in accuracy than when predicting the behavior of the user correspondingly to a periodically occurring situation, therefore the electronic device cannot guarantee a reliability of information for the user.

Reference is made to US 2014/0335490A1, which discloses a behavior modification system including a network of components that interact to collect various data and provide user feedback. The network may include a personal device, an Internet-enabled storage device and a hub capable of receiving communications from the personal device and communicating to the storage device. The personal device may include bio-impedance measurement circuitry, an accelerometer and a processor for determining energy expenditure based on data from the accelerometer(s). The system may include a smart hub capable of routing communications between various components within the system. The hub may include different transceivers for different communication protocols. The system may incorporate a low-power RF wake-up system. The system may include bio-impedance measurement circuitry that is reconfigurable to function as an alternative type of sensor. There is also disclosed a method for measuring bio-resonance and a method for determining caloric intake from body composition and caloric expenditure.

### SUMMARY

According to various exemplary embodiments, a method for operating in a wearable device is provided. The method may include determining an acquisition condition for acquiring biometric information of a user of the wearable device, based on movement information acquired by a movement detection unit, wherein determining the acquisition condition for acquiring biometric information of the user comprises: determining whether a state of the wearable device is stationary for at least a previously designated time period based on acquired movement information; if the state of the wearable device is stationary for at least the designated time period, acquiring location information of the user; determining whether the acquired location of the user corresponds with a designated situation, wherein the designated situation is a situation corresponding to schedule information of the user stored in the memory, wherein the schedule information comprises position and time information of the user; and if the acquired location does not correspond with a designated situation, acquiring the biometric information of the user. The method further comprises: storing, in at least one of the memory and an external server, the determined acquisition condition and the biometric information of the user; comparing the stored biometric information of the user with a predesignated setting value; and controlling the wearable device or another electronic device communicatively coupled with the wearable device in accordance with the comparison result.

According to various exemplary embodiments, a wearable device is provided. The wearable device may include a movement detection unit, a position detection unit, a wireless communication unit, a biometric information detection unit, a memory, and a processor. The processor is configured to determine an acquisition condition for acquiring biometric information of a user of the wearable device based on movement information acquired by the movement detection unit, wherein determining the acquisition condition for acquiring biometric information of the user comprises: determining whether a state of the wearable device is stationary for at least a previously designated time period based on acquired movement information; if the state of the wearable device is stationary for at least the designated time period, acquiring location information of the user; determining whether the acquired location of the user corresponds with a designated situation, wherein the designated situation is a situation corresponding to schedule information of the user stored in the memory wherein the schedule information comprises position and time information of the user; and if the acquired location does not correspond with a designated situation, acquiring the biometric information of the user. The processor is further configured to: store, in at least one of the memory and an external server, the determined acquisition condition and the biometric information of the user; compare the stored biometric information of the user with a predesignated setting value; and control the wearable device or another electronic device communicatively coupled with the wearable device in accordance with the comparison result.

### BRIEF DESCRIPTION OF THE DRAWINGS

The present disclosure will become more apparent from the following detailed description when taken in conjunction with the accompanying drawings in which:
FIG. 1 illustrates a network environment including an electronic device according to various example embodiments;
FIG. 2 illustrates a flow of an operation corresponding to a specific situation in an electronic device according to various example embodiments;
FIG. 3 illustrates a flow of an operation according to wearing in an electronic device according to various example embodiments;
FIG. 4 illustrates a flow of an operation according to an exceptional situation in an electronic device according to various example embodiments;
FIG. 5 illustrates a flow of an operation of utilizing acquired information in case of a valid event in an electronic device according to various example embodiments;
FIG. 6 is a diagram related with an event stored in an electronic device according to various example embodiments;
FIG. 7 is a diagram related with an event not stored in an electronic device according to various example embodiments;
FIG. 8A is a diagram illustrating an operation of determining a user situation dependent on blood sugar variation in an electronic device according to various example embodiments;
FIG. 8B is a diagram illustrating an operation of analyzing detected biometric information in an electronic device according to various example embodiments;
FIG. 9 is a diagram illustrating an operation of determining a user's state based on detected biometric information in an electronic device according to various example embodiments;
FIG. 10 is a diagram illustrating an operation of determining a situation in which a user is placed based on acquired information in an electronic device according to various example embodiments;
FIG. 11 is a diagram illustrating an operation of performing a designated operation based on a user's position in an electronic device according to various example embodiments;
FIG. 12 is a diagram illustrating an operation dependent on user's biometric information variation in an electronic device according to various example embodiments;
FIG. 13 is a diagram illustrating an operation of displaying a user situation that is determined based on biometric information in an electronic device according to various example embodiments;
FIG. 14 is a diagram illustrating an operation dependent on user's biometric information variation in an electronic device according to various example embodiments;
FIG. 15 is a diagram illustrating an operation of controlling another electronic device based on user's biometric information in an electronic device according to various example embodiments;
FIG. 16 is a diagram illustrating an operation of performing a designated operation based on user's biometric information at a specific time point in an electronic device according to various example embodiments;
FIG. 17 is a diagram illustrating an operation of changing a designated condition based on user's biometric information in an electronic device according to various example embodiments; and
FIG. 18 is a diagram illustrating an operation of performing an operation of a designated schedule based on log data in an electronic device according to various example embodiments.

### DETAILED DESCRIPTION

Hereinafter, various embodiments of the present disclosure will be described with reference to the accompanying drawings. However, it should be understood that there is no intent to limit the present disclosure to the particular forms disclosed herein; rather, the present disclosure should be construed to cover various modifications, equivalents, and/or alternatives of embodiments of the present disclosure. In describing the drawings, similar reference numerals may be used to designate similar constituent elements.

In the present disclosure, the expression "have", "may have", "include" or "may include" refers to existence of a corresponding feature (e.g., numerical value, function, operation, or components such as elements), and does not exclude existence of additional features.

In the present disclosure, the expression "A or B," "at least one of A or/and B," or "one or more of A or/and B" may include all possible combinations of the items listed. For example, the expression "A or B," "at least one of A and B," or "at least one of A or B" refers to all of (1) including at least one A, (2) including at least one B, or (3) including all of at least one A and at least one B.

The expression "a first," "a second," "the first," or "the second" used in various embodiments of the present disclosure may modify various components regardless of the order and/or the importance but does not limit the corresponding components. For example, a first electronic device and a second electronic device may indicate different user devices, regardless of order or importance thereof. For example, a first element may be interchangeably referred to as a second element, and similarly, a second element may be interchangeably referred to as a first element without departing from the present disclosure.

It should be understood that when an element (e.g., a first element) is referred to as being (operatively or communicatively) "connected" or "coupled" to another element (e.g., second element), it may be directly connected or coupled directly to the other element. In such a situation, alternatively, any other element (e.g., third element) may be interposed between them. In certain embodiments, it may be understood that when an element (e.g., first element) is referred to as being "directly connected," or "directly coupled" to another element (second element), there are no element (e.g., third element) interposed between them (while there can be a connecting element, such as an adhesive or a connector between them).

The expression "configured to" used in the present disclosure may be interchangeably used with, for example, "suitable for," "having the capacity to," "designed to," "adapted to," "made to," or "capable of," depending on the context. The term "configured to" may not necessarily imply "specifically designed to" in hardware. Alternatively, in some situations, the expression "device configured to" may mean that the device, together with other devices or components, "is able to." For example, the phrase "processor adapted (or configured) to perform A, B, and C" may mean a dedicated processor (e.g. embedded processor) for performing the corresponding operations or a generic-purpose processor (e.g., central processing unit (CPU) or application processor (AP)) that can perform the corresponding operations by executing one or more software programs stored in a memory device.

The terms used herein are merely for the purpose of describing particular embodiments and are not intended to limit the other embodiments. As used herein, singular forms may include plural forms as well unless the context clearly indicates otherwise. Unless defined otherwise, all terms used herein, including technical and scientific terms, have the same meaning as those commonly understood by a person skilled in the art to which the present disclosure pertains. Such terms as those defined in a generally used dictionary may be interpreted to have the meanings equal to the contextual meanings in the relevant field of art, and are not to be interpreted to have ideal or excessively formal meanings unless clearly defined in the present disclosure. In some cases, even the term defined in the present disclosure should not be interpreted to exclude embodiments of the present disclosure.

An electronic device according to various embodiments of the present disclosure may include at least one of, for example, a smart phone, a tablet Personal Computer (PC), a mobile phone, a video phone, an electronic book reader (e-book reader), a desktop PC, a laptop PC, a netbook computer, a workstation, a server, a Personal Digital Assistant (PDA), a Portable Multimedia Player (PMP), a MPEG-1 audio layer-3 (MP3) player, a mobile medical device, a camera, and a wearable device. According to various embodiments, the wearable device may include at least one of an accessory type (e.g., a watch, a ring, a bracelet, an anklet, a necklace, a glasses, a contact lens, or a Head-Mounted Device (HMD)), a fabric or clothing integrated type (e.g., an electronic clothing), a body-mounted type (e.g., a skin pad, or tattoo), and a bio-implantable type (e.g., an implantable circuit).

According to some embodiments, the electronic device may be a home appliance. The home appliance may, for example, include at least one of a television, a digital video disk (DVD) player, an audio player, a refrigerator, an air conditioner, a cleaner, an oven, a microwave oven, a washing machine, an air purifier, a set-top box, a home automation control panel, a TV box (e.g., HomeSync^{™} of Samsung, Apple TV^{™}, or Google TV^{™}), a game console (e.g., Xbox^{™}, PalyStationTM), an electronic dictionary, an electronic key, a camcorder, and an electronic frame.

According to another embodiment, the electronic device may include at least one of various medical devices (e.g., various portable medical measuring devices (a blood glucose measuring device, a heart rate measuring device, a blood pressure measuring device, a body temperature measuring device, etc.), a Magnetic Resonance Angiography (MRA), a Magnetic Resonance Imaging (MRI), a Computed Tomography (CT) machine, and an ultrasonic machine), a navigation device, a Global Navigation Satellite System (GNSS) receiver, an Event Data Recorder (EDR), a Flight Data Recorder (FDR), a Vehicle Infotainment Devices, an electronic devices for a ship (e.g., a navigation device for a ship, and a gyro-compass), avionics, security devices, an automotive head unit, a robot for home or industry, an automatic teller's machine (ATM) in banks, point of sales (POS) in a shop, or internet device of things (e.g., a light bulb, various sensors, electric or gas meter, a sprinkler device, a fire alarm, a thermostat, a streetlamp, a toaster, a sporting goods, a hot water tank, a heater, a boiler, etc.).

According to some embodiments, the electronic device may include at least one of a part of furniture or a building/structure, an electronic board, an electronic signature-receiving device, a projector, and various kinds of measuring instruments (e.g., a water meter, an electric meter, a gas meter, and a radio wave meter). The electronic device according to various embodiments of the present disclosure may be a combination of one or more of the aforementioned various devices. The electronic device according to some embodiments of the present disclosure may be a flexible device. Further, the electronic device according to an embodiment of the present disclosure is not limited to the aforementioned devices, and may include a new electronic device according to the development of technology.

Hereinafter, an electronic device according to various embodiments will be described with reference to the accompanying drawings. As used herein, the term "user" may indicate a person who uses an electronic device or a device (e.g., an artificial intelligence electronic device) that uses an electronic device.

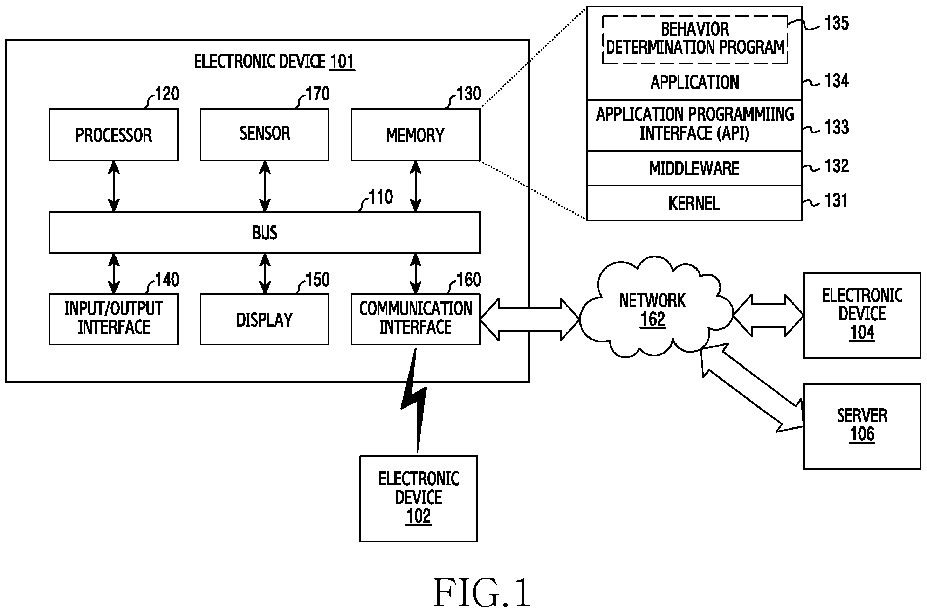

FIG. 1 is a diagram illustrating a network environment including an electronic device according to various embodiments of the present disclosure.

Referring to FIG. 1, the electronic device 101 may include at least one of a bus 110, a processor 120, a memory 130, an input/output (I/O) interface 140, a display 150, a communication interface 160 and a sensor 170.

The bus 110 may, for example, be a circuit for connecting the above-described elements with each other, and transferring communication (e.g., a control message) between the above-described elements.

The processor 120 may receive, for example, an instruction from the above-described other elements (e.g., the memory 130, the I/O interface 140, the display 150, or the communication interface 160, etc.) via the bus 110, decipher the received instruction, and execute an operation or a data process corresponding to the deciphered instruction.

The processor 120 may be included in the electronic device 101 to perform a specified function of the electronic device 101. According to an embodiment of the present disclosure, the processor 120 may include one or more application processors (APs) and one or more microcontroller units (MCUs). According to another embodiment of the present disclosure, the processor 120 may include one or more MCUs as applications, or may be functionally connected to one or more MCUs. In FIG. 1, the APs and the MCUs may be included in one integrated circuit (IC) package, or may be separately configured to be included in different IC packages, respectively. According to an embodiment of the present disclosure, the MCUs may also be included in an IC package of the APs so as to be configured as one IC package together with the APs. Although the processor 120 is illustrated as including the APs or the MCUs, it is nothing more than an embodiment for clear understanding, and it is apparent that the processor 120 may also perform the operations of the APs and/or the MCUs.

The APs may control a plurality of hardware or software elements connected thereto and may perform processing and operations on various types of data including multimedia data by driving an operating system (OS) or application programs (or applications). The APs may be embodied as, for example, a system on chip (SoC). According to an embodiment of the present disclosure, the processor 120 may further include a graphics processing unit (GPU) (not illustrated).

The MCUs may be processors configured to perform specified operations. According to an embodiment of the present disclosure, the MCUs may acquire sensing information through one or more specified motion sensors (e.g., a gyro sensor, an acceleration sensor, and a geomagnetic sensor), compare the acquired sensing information, and determine the respective operating states of the specified sensors with reference to a database of the electronic device 101. According to an embodiment of the present disclosure, the MCUs may further include a part of the sensor 170.

According to an embodiment of the present disclosure, the APs or the MCUs may load instructions or data received from at least one of non-volatile memories or other elements connected thereto in volatile memories, and may process the loaded instructions or data. Furthermore, the APs or the MCUs may store data received from or generated by at least one of the other elements in the non-volatile memories.

The memory 130 may store commands or data (e.g., a reference pattern or a reference touch area) associated with one or more other components of the electronic device 101. According to an embodiment of the present disclosure, the memory 130 may store software and/or a program. For example, the program may include a kernel 131, a middleware 132, an application programming interface (API) 133, an application program 134, or the like. At least some of the kernel 131, the middleware 132, and the API 133 may be referred to as an OS.

The kernel 131 may control or manage system resources (e.g., the bus 110, the processor 120, or the memory 130) used for performing an operation or function implemented by the other programs (e.g., the middleware 132, the API 133, or the applications 134). Furthermore, the kernel 131 may provide an interface through which the middleware 132, the API 133, or the applications 134 may access the individual elements of the electronic device 101 to control or manage the system resources.

The middleware 132, for example, may function as an intermediary for allowing the API 133 or the applications 134 to communicate with the kernel 131 to exchange data. In addition, the middleware 132 may process one or more task requests received from the applications 134 according to priorities thereof. For example, the middleware 132 may assign priorities for using the system resources (e.g., the bus 110, the processor 120, the memory 130, or the like) of the electronic device 101, to at least one of the applications 134. For example, the middleware 132 may perform scheduling or loading balancing on the one or more task requests by processing the one or more task requests according to the priorities assigned thereto.

The API 133 is an interface through which the applications 134 control functions provided from the kernel 131 or the middleware 132, and may include, for example, at least one interface or function (e.g., instruction) for file control, window control, image processing, or text control.

The application (or the processor) 134 may include a short message service (SMS)/multimedia message service (MMS) application, an electronic mail (e-mail) application, a calendar application, an alarm application, a health care application (e.g., an application measuring momentum or blood sugar, etc.), or an environment information application (e.g., an application providing air pressure, humidity or temperature information, etc.), etc. The application (or the processor) 134 may be an application related with information exchange between the first electronic device 101 and an external electronic device (e.g., a second electronic device 102 or an electronic device 103). The application related with the information exchange may, for example, include a notification relay application for relaying specific information to the external electronic device or a device management application for managing the external electronic device.

In examples of the aforementioned applications, the notification relay application may include a function of relaying notification information generated in another application (e.g., the SMS/MMS application, the e-mail application, the health care application or the environment information application, etc.) of the first electronic device 101, to the external electronic device (e.g., the electronic device 103). Additionally or alternatively, the notification relay application may, for example, receive notification information from the external electronic device (e.g., the electronic device 103) and provide the received notification information to a user.

The device management application may, for example, manage (e.g., install, delete or update) a function (e.g., turn-on / turn-off of the external electronic device itself (or some constituent components) or adjustment of a brightness (or resolution) of a display) of at least a part of the external electronic device (e.g., the electronic device 103 communicating with the first electronic device 101, an application operating in the external electronic device, or a service (e.g., a telephony service or a message service) provided from the external electronic device.

Similarly, in case where the external electronic device is the mobile medical equipment, the application 134 may include an application related with health care. According to one example embodiment, the application 134 may include at least one of an application designated to the electronic device 101 or an application received from the external electronic device (e.g., the server 106 or the electronic device 104). A behavior determination program 135 may be included and provided in the application 134, or may be stored as a separate program in the memory 130.

The behavior determination program 135 may acquire device situation information of the electronic device 101 through the sensor unit 170, and check a specific situation based on the device situation information. In case where it is not an event designated to the specific situation, the behavior determination program 135 may acquire user's biometric information through the sensor unit 170. In case where the acquired biometric information satisfies a designated condition, the behavior determination program 135 may store the specific situation and the biometric information.

According to one example embodiment, the behavior determination program 135 may include, in device situation information, at least one information among a movement speed of the electronic device 101, an acceleration thereof, a movement direction thereof, a height thereof, an impulse applied to the electronic device 101, a temperature and air pressure around the electronic device 101, and a current time. According to one example embodiment, the behavior determination program 135 may determine, as a case where it is not an event designated to a specific situation, a case where it is not an event designated to schedule information or a case where it is not an event repeatedly performed. According to one example embodiment, the behavior determination program 135 may include, in user's biometric information, at least one information among a blood pressure of a user, a heartbeat (or pulse), a blood flow, a blood vessel state, a body temperature, a body composition, a pupil (iris and/or eye pupil) state, a calorie expenditure, an oxygen saturation, breathing, blood sugar, an electromyogram, an electroencephalogram, an electrocardiogram, an ElectroDermal Activity (EDA), and a Galvanic Skin Response (GSR).

According to one example embodiment, the behavior determination program 135 may acquire user's biometric information in a state in which the electronic device 101 is attached to or worn on a part of the body of a user. According to one example embodiment, when the electronic device 101 measures a body composition in a state in which the electronic device 101 is worn on the part of the body of the user, the electronic device 101 may use Bioelectrical Impedance Analysis (BIA). According to one example embodiment, in case where it is repeated at a designated count or more that biometric information acquired in a specific situation satisfies a designated condition, the behavior determination program 135 may store an event corresponding to the specific situation and the biometric information.

According to one example embodiment, the behavior determination program 135 may perform an operation corresponding to a designated condition. According to one example embodiment, the behavior determination program 135 may perform the operation corresponding to the designated condition based on a specific situation. According to one example embodiment, the behavior determination program 135 may detect a variation of user's blood sugar based on acquired biometric information. According to one example embodiment, the behavior determination program 135 may measure the user's blood sugar after the lapse of a designated time from a time point of detecting the variation of the user's blood sugar.

The behavior determination program 135 may check user situation information at a specific time point through a medical sensor and perform a function corresponding to the specific time point and the user situation information based on log data. The behavior determination program 135 may determine, as the specific time point, at least one of a time point periodically detecting a designated operation, a time point that is set based on a user input, and payment information. Through the log data, the behavior determination program 135 may process setting information by the user input, and information acquired in the electronic device 101 before the specific time point. The behavior determination program 135 may process, as acquired information, information about an operation periodically carried out by a user.

The behavior determination program 135 may include and process user's biometric information in user situation information. The behavior determination program 135 may process, as the user's biometric information, information about at least one of a heart rate (or a cardiac rate), blood sugar, a blood pressure, and a body temperature. The behavior determination program 135 may check, as a specific time point, an alarm time that is set to the electronic device 101, and change the set alarm time based on the user situation information. The behavior determination program 135 may detect a variation of user's blood sugar by the user situation information and, based on a user input, check at least one information among eating or non-eating and a meal menu. The behavior determination program 135 may check user's information and at least one information of another user, and change a scheme of execution of a designated function based on the at least one information of the another user.

The I/O interface 140 may forward an instruction or data inputted from a user through an I/O device (e.g., various sensors such as an acceleration sensor and a gyro sensor and/or a device such as a keyboard or a touch screen), for example, to the processor 120, the memory 130 or the communication interface 160 through the bus 110. For example, the I/O interface 140 may provide data about a user's touch inputted through a touch screen, to the processor 120. Also, the I/O interface 140 may, for example, output an instruction or data received from the processor 120, the memory 130 and the communication interface 160 through the bus 110, through an output device (e.g., a speaker or the display 150). For example, the I/O interface 140 may output voice data processed by the processor 120, to a user through the speaker.

The display 150 may display various information (e.g., multimedia data or text data, etc.) to a user. Also, the display 150 may include a touch screen for inputting an instruction by touching or proximity touching an input means to a display.

The communication interface 160 (e.g., the communication module 220) may connect a communication between the first electronic device 101 and the external device (e.g., the electronic device 103 or the server 106). For example, the communication interface 160 may connect to a network 162 through wireless communication or wired communication, and communicate with the external device. The wireless communication may include, for example, at least one of short-range wireless communication such as WiFi, Bluetooth (BT), near field communication (NFC), and GPS, or cellular communication (e.g., long term evolution (LTE), LTE-advanced (LTE-A), code division multiple access (CDMA), wideband CDMA (WCDMA), universal mobile telecommunications system (UMTS), wireless broadband (WiBro), or global system for mobile communications (GSM)). The wired communication may include, for example at least one of a universal serial bus (USB), a high definition multimedia interface (HDMI), a recommended standard-232 (RS-232), or a plain old telephone service (POTS).

The electronic device 101 may include a wireless communication unit includes at least one communication module available for the wireless communication. The wired communication may, for example, include at least one of a Universal Serial Bus (USB), a High Definition Multimedia Interface (HDMI), a Recommended Standard 232 (RS-232) or a Plain Old Telephone Service (POTS). The electronic device 101 may include a wired communication unit includes at least one communication module available for the wired communication.

The sensor unit 170 may meter a physical quantity and detect an activation state of the electronic device 101, and convert metered or detected information into an electric signal. The sensor unit 170 may, for example, include at least one of a gesture sensor, a gyro sensor, an air pressure sensor, a magnetic sensor, an acceleration sensor, a grip sensor, a proximity sensor, a color sensor (e.g., a Red, Green, Blue (RGB) sensor), a temperature/humidity sensor, an illuminance sensor or UltraViolet (UV) sensor, and a positioning sensor (e.g., a GPS). Additionally or alternatively, the sensor unit 170 may, for example, include at least one of medical sensors such as an E-nose sensor, an Electromyography (EMG) sensor, an Electroencephalogram (EEG) sensor, an Electrocardiogram (ECG) sensor, an Infrared (IR) sensor, an iris scan sensor or finger scan sensor, a blood pressure measurement sensor, a blood flow measurement sensor, a blood vessel state measurement sensor, and a body composition measurement sensor.

The sensor unit 170 may further include a control circuit for controlling at least one or more sensors belonging therein. According to various example embodiments, the sensor unit 170 may acquire information (hereinafter, biometric information) about a medical state of a user who wears the electronic device 101 through at least one medical sensor. According to one example embodiment, in case where the electronic device 101 includes a perspiration sensor as the medical sensor, the electronic device 101 may acquire information such as user's blood sugar and a body composition, from perspiration produced from the body of the user.

According to various example embodiments, when the electronic device 101 measures user's blood sugar, the electronic device 101 may use a blood sugar measurement sensor of an ultrasonic scheme, an optical scheme, without being limited to the perspiration sensor. In case where the electronic device 101 includes a temperature sensor as the medical sensor, the electronic device 101 may check a body temperature of the user through a part of the body of the user who comes in contact with the electronic device 101. In case where the electronic device 101 includes a heart rate measurement sensor as the medical sensor, the electronic device 101 may detect a pulse of the user through a part of the body of the user who comes in contact with the electronic device 101, and check a heart rate of the user.

In case where the electronic device 101 includes a blood pressure measurement sensor as the medical sensor, the electronic device 101 may measure a blood pressure of a user through a part of the body of the user who comes in contact with the electronic device 101. According to various example embodiments, when the electronic device 101 checks and/or measures various biometric information of the user through the aforementioned medical sensor, the electronic device 101 may acquire the biometric information of the user checked and/or measured from the medical sensor which is attached to a part of the body of the user independently of the electronic device 101, without being limited to coming in contact to the part of the body of the user and checking and/or measuring through the medical sensor. According to one example embodiment, the electronic device 101 may be a wearable device worn on the user's wrist and, when the electronic device 101 acquires a user's heart rate, the electronic device 101 may acquire, by network communication, the user's heart rate from a heart rate measurement sensor that is attached to a position (e.g., near the heart) capable of checking and/or measuring a user's heartbeat.

According to various example embodiments, when implementing the sensor unit 170, the electronic device 101 may implement the sensor unit 170 by one module, and may implement the sensor unit 170 by at least two or more modules as well. According to one example embodiment, the electronic device 101 may implement the sensor unit 170 by a plurality of modules such as a movement detection unit, a position detection unit, and a biometric information detection unit.

According to an embodiment of the present disclosure, the network 162 may be a telecommunications network. The telecommunications network may include at least one of a computer network, the Internet, internet of things (IoT), or a telephone network. According to an embodiment of the present disclosure, a protocol (e.g., a transport layer protocol, a data link layer protocol, or a physical layer protocol) for a communication between the first electronic device 101 and the external device may be supported in at least one of the applications 134, the API 133, the middleware 132, the kernel 131, or the communication interface 160.

According to an embodiment of the present disclosure, the server 106 may support driving of the first electronic device 101 by performing at least one operation among operations (or functions) implemented in the first electronic device 101. For example, the server 106 may include a server module (e.g., a server controller or a server processor, not shown) that is able to support the processor 120 controlling to perform various example embodiments of the present disclosure described later below in the electronic device 101 or a specific module designated to perform various example embodiments. For example, the server module may include at least one constituent element of the processor 120 or the specific module, and perform (e.g., act for) at least one operation among operations carried out by the processor 120 or the specific module. According to various example embodiments, the server module may be denoted as the server 106 of FIG. 1.

According to various example embodiments, the electronic device 101 may process an input generated in the electronic device 101 based on a database stored in the memory 130. For example, the input generated in the electronic device 101 may be a variety of inputs detected through the display 150 (e.g., it may be configured to include a touch screen), at least one physical key (or button) included in the electronic device 101 and/or at least one sensor constituting the sensor unit 170 of the electronic device 101.

According to various example embodiments, by using at least one sensor included in (or coupled to) the sensor unit 170, the electronic device 101 may acquire device situation information of the electronic device 101, or biometric information of a user who wears the electronic device 101 in case where the electronic device 101 is worn on a part of the body of the user. According to one example embodiment, when the electronic device 101 acquires the device situation information, the electronic device 101 may acquire at least one of information of a movement of the electronic device 101, for example, a movement speed, an acceleration, a movement direction, a height, an impulse applied to the electronic device 101, a temperature and an air pressure around the electronic device 101, and a current time of the electronic device 101, etc. Further, when the electronic device 101 acquires user's biometric information, the electronic device 101 may acquire at least one of information of a blood pressure of the user, a heartbeat (or pulse), a blood flow, a blood vessel state, a body temperature, a body composition, a pupil (iris and/or eye pupil) state, a calorie expenditure, an oxygen saturation, an electrocardiogram, an electroencephalogram, an electromyogram, an EDA, a GSR, a BIA, etc.

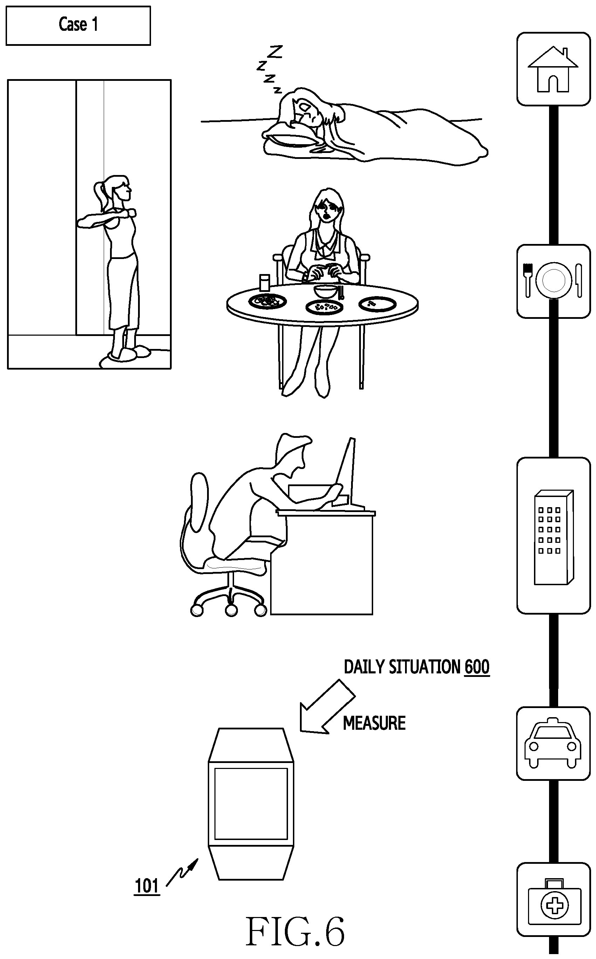

According to various example embodiments, when the electronic device 101 acquires device situation information of the electronic device 101 and/or biometric information of a user, the electronic device 101 may acquire them based on a schedule stored in schedule information of the electronic device 101, or may acquire them based on a user input. According to one example embodiment, the electronic device 101 may acquire the device situation information of the electronic device 101 and/or the biometric information of the user, based on a designated position or designated time being based on schedule information stored in the memory 130. Below, the electronic device 101 may define, as a daily situation (e.g., a daily situation 600 of FIG. 6), a situation of acquiring the device situation information of the electronic device 101 and/or the biometric information of the user based on the schedule information of the electronic device 101.

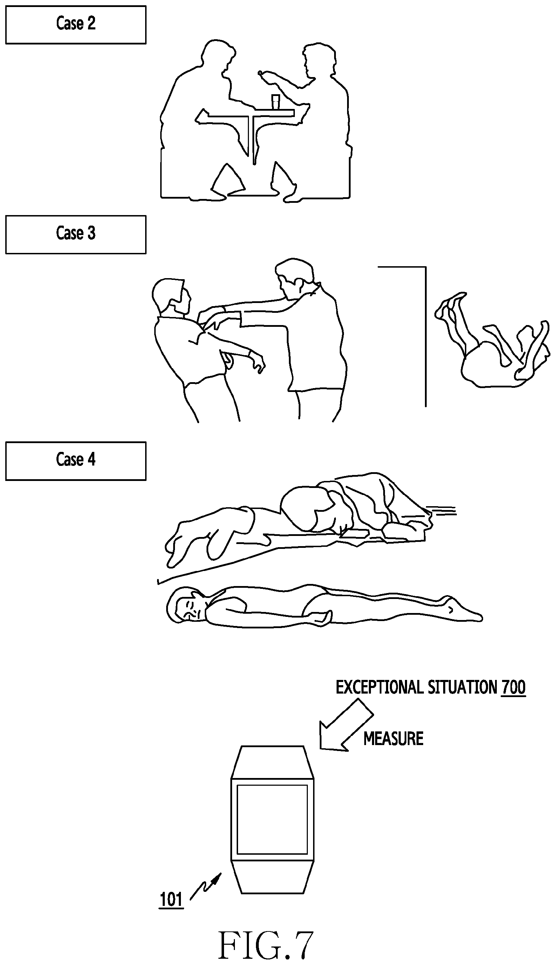

According to one example embodiment, when the electronic device 101 acquires device situation information and/or biometric information, the electronic device 101 may acquire the user's biometric information in a specific situation, without being based on schedule information. For example, when the electronic device 101 acquires the device situation information, some sensors may acquire the device situation information, without being based on the schedule information of the electronic device 101. According to one example embodiment, at least one sensor among sensors acquiring (or measuring) movement information of the electronic device 101 such as an acceleration sensor included in the electronic device 101, a speed sensor, an inclination sensor, a positioning sensor (e.g., a GPS), and a gyro sensor (or gyroscope) may acquire the movement information of the electronic device 101 by periods (e.g., at a designated time interval). In case where the periodically acquired device situation information satisfies a specific situation, the electronic device 101 may acquire the user's biometric information. In case where the acquired user's biometric information satisfies a designated condition, the electronic device 101 may store the specific situation and the acquired user's biometric information. The electronic device 101 may define, as exceptional situations (e.g., exceptional situations 700 of FIG. 7), a situation in which the device situation information satisfies the specific situation and a situation in which the user's biometric information satisfies the designated condition as mentioned above.

According to various example embodiments, the electronic device 101 may build a database for a user situation based on device situation information of the electronic device 101 and biometric information of a user which are acquired in a daily situation and an exceptional situation. Further, the electronic device 101 may analyze a user's action or situation correspondingly to the database and a situation occurring in the electronic device 101, and may provide information such as a user's living guide based on analyzed information.

According to various example embodiments, the electronic device 101 may perform an operation such as user identification (authentication), health state (including emotion state) checking, momentum determination, and life logging (upon event occurrence, determining the type of an event and recording a user state according to the event) based on the acquired biometric information.

According to various example embodiments, the electronic device 101 may repeatedly measure (regularly measure) the regular daily situation 600 at a designated time point based on a condition that a user previously designates. The electronic device 101 may tag a measurement condition and an environment of that time, etc. to the measured biometric information. For example, the regular measurement condition may be set based on a user input such as once per twenty minutes at sleep, soon after getting up in the morning (e.g., a time point of wearing in case where the electronic device 101 is taken off), once every two hours for week days, once every three hours for a holiday, etc. The electronic device 101 may store the tagged information (e.g., biometric information and/or device situation information) in the memory 130 of the electronic device 101 or at least one another electronic device (e.g., server) coupled with the electronic device 101 by network communication.

According to various example embodiments, measuring the exceptional situation 700 may mean that the electronic device 101 measures biometric information at occurrence of a specific event excepting regular measurement. For example, the electronic device 101 may tag a measurement condition and an environment of that time, etc. to the measured biometric information. For example, an event detection condition may include an exceptional measurement condition such as a place alteration (movement between floors, movement between buildings, movement between interiors or exteriors), a sudden movement (acceleration generation of a reference value or more), a sudden height variation in the same place, non-detecting of movement for a specific time or more, meeting with a specific person (determined by device searching and/or pairing), etc.

For example, to detect the exceptional measurement condition, the sensor unit 170 (e.g., a motion sensor such as an acceleration sensor, a height sensor, and a clinometer sensor) may be in an Always-On state. Or, the sensor unit 170 (e.g., a GPS of much power consumption) may turn On/Off on a basis of a variation of a coupling state (connect, search, RSSI) of network communication. For example, the GPS may turn On, in case where an available Access Point (AP) has gone as the electronic device 101 moves from the interior to the exterior to gradually weaken a signal strength with the AP and cut a coupling between the electronic device 101 and the AP or in case where getting in a car and/or high-speed movement is detected.

According to various example embodiments, the electronic device 101 may store a measurement result and a user's feedback of the result in a database, or store them in a server. For example, the stored information may be used for later user's checking and utilizing (e.g., utilizing such as operation mode determination upon similar situation occurrence, submission upon medical diagnosis, etc.).

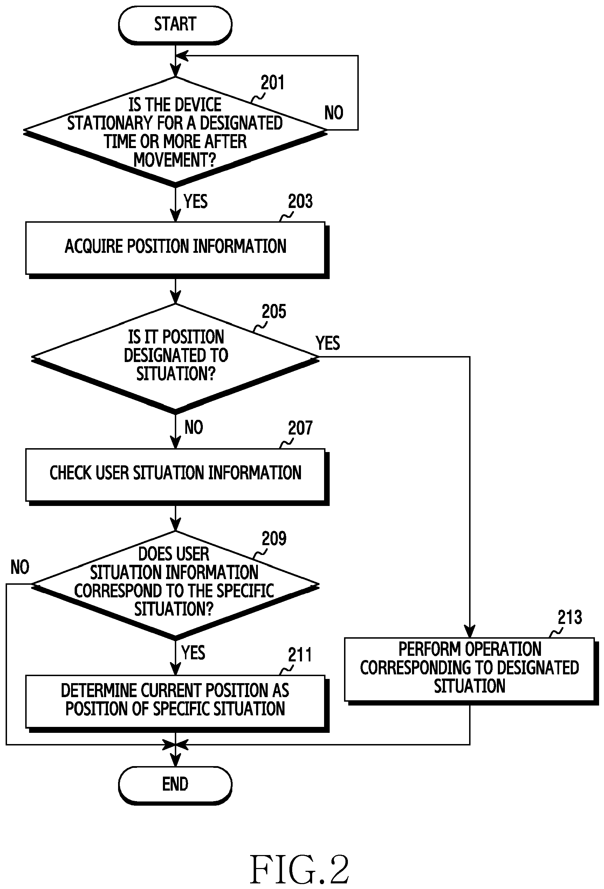

FIG. 2 illustrates a flow of an operation corresponding to a specific situation in an electronic device according to various example embodiments.

In operation 201, the electronic device 101 may determine if it is in a state of being stationary for a designated time or more in a place where the electronic device 101 is located based on acquired movement information. According to one example embodiment, the electronic device 101 may check if it is stationary for a designated time (e.g., ten minutes) or more without moving out of a designated area or space (e.g., within a three-meter radius). Further, when the electronic device 101 is to determine that is has been stationary for the designated time or more in the designated space where the electronic device 101 is located, the electronic device 101 may detect this state by checking whether it has been stationary for the designated time (e.g., ten minutes) or more moving out of the designated space or area (e.g., three-meter radius), after moving a designated distance (e.g., ten meters) or more. The movement may indicate, for example, movements of various degrees such as, moving between floors in a single building, or movement between buildings.

In operation 203, the electronic device 101 may acquire position information. According to various example embodiments, the electronic device 101 may acquire the position information of the electronic device 101 using a positioning sensor (e.g., a GPS), or may acquire the position information of the electronic device 101 based on a traffic line of the electronic device 101 acquired using the sensor 170. Or, the electronic device 101 may acquire the positon information of the electronic device 101 from an available access point that is determined through the communication interface 160.

In operation 205, the electronic device 101 may check if a specific situation has been designated corresponding to the position information for which the electronic device 101 is stationary, based on schedule information. According to one example embodiment, the electronic device 101 may determine if the specific situation stored in the schedule information exists, and whether it corresponds to the acquired position information and/or a time. For example, the electronic device 101 may be in a state corresponding to a scheduled lunch as indicated by the schedule information to occur during a time range of 12:30 - 13:30, and may include a plurality of various position information (e.g., geographic locations) as indicated by the schedule information to correspond to the lunch time 12:30-13:30. Here, the plurality of various position information stored in the schedule information corresponding to the lunch time 12:30 ~ 13:30 may indicate places and venues which corresponding to lunch (e.g., restaurants) designated by a user input.

Thus, in operation 205, as indicated above, the electronic device 101 may determine if a schedule corresponding to acquired position information and/or time information exists. For example, in case where the position information acquired in the electronic device 101 is included in a lunch place stored in schedule information of the electronic device 101 and the time of the electronic device 101 is included in a time range of a lunch time 12:30-13:30 stored in the schedule information, the electronic device 101 may determine a user situation as a specific situation (e.g., a lunch). In case where determining the user situation as the specific situation, the electronic device 101 may perform operation 213.

According to various example embodiments, in case where acquired position information or time information of the electronic device 101 does not match with a schedule stored in schedule information of the electronic device 101, the electronic device 101 may perform operation 207. According to one example embodiment, in case where the position information acquired in the electronic device 101 is a lunch place indicated by the schedule information of the electronic device 101, but the presently-indicated time is not within the lunch time 12:30-13:30 indicated by the schedule information, the electronic device 101 may perform operation 207. According to another example embodiment, in case where the time is within the indicated lunch time 12:30-13:30 from the schedule information, but the acquired position information indicated that the electronic device 101 is not located the lunch place as indicated by the schedule information of the electronic device 101, the electronic device 101 may perform operation 207.

In operation 207, the electronic device 101 may check user situation information. According to various example embodiments, when acquiring the user situation information, the electronic device 101 may acquire device situation information of the electronic device 101 and/or biometric information of a user. For example, in case where the acquired position information is a coordinate, the electronic device 101 may check place information corresponding to coordinate information of the electronic device 101 through the Internet or another electronic device (e.g., a server 106) coupled by network communication. For example, the place information may be at least a part of information among a building name, a shop name, the remains, a transportation, etc. that are associated with the coordinate.

For example, the biometric information of the user may include at least one of information of a blood pressure of the user, a heartbeat (or pulse), a blood flow, a blood vessel state, a body temperature, a body composition, an eye pupil (iris and/or pupil) state, a calorie expenditure, an oxygen saturation, breathing, blood sugar, an electrocardiogram, an electroencephalogram, an electromyogram, an EDA, a GSR, a BIA, etc.

In operation 209, the electronic device 101 may determine if a user situation corresponds to a predictable specific situation, based on the checked user situation information. Here, the predictable specific situation may represent various situations stored to determine the user situation with reference to a database of the electronic device 101 based on the user situation information acquired in the electronic device 101.

According to one example embodiment, in case where position information of the electronic device 101 or place information checked as the position information is a meal place (e.g., a restaurant), and/or checked biometric information of a user is determined to be "food intake," the electronic device 101 may determine the user situation as a "food intake situation." For example, the electronic device 101 may check a variation of user's blood sugar. In case where the electronic device 101 checks the variation of the user's blood sugar, the electronic device 101 may determine that the user is consuming food, and may further determine the type of food that the user is consuming by means of a blood sugar variation pattern, the position information of the electronic device 101, or the place information checked as the position information.

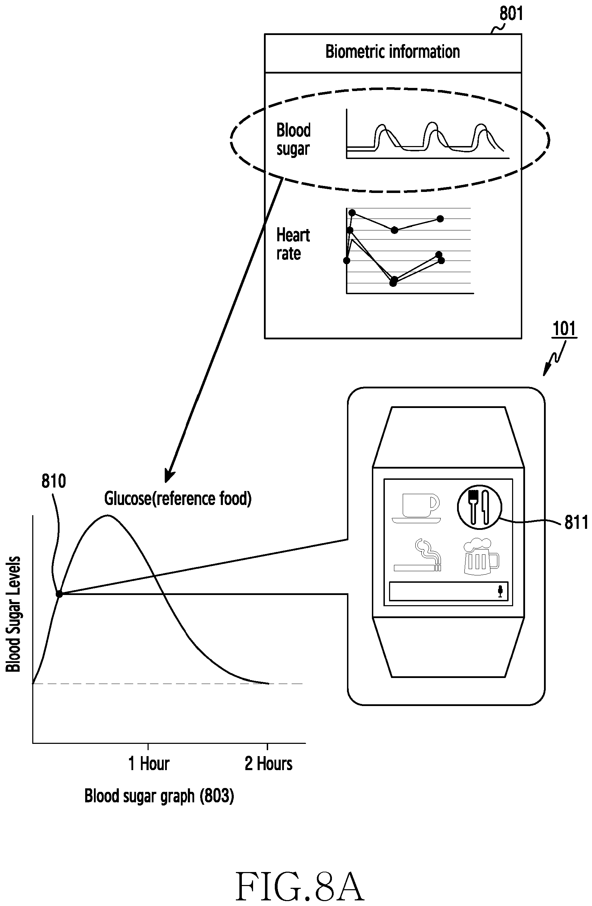

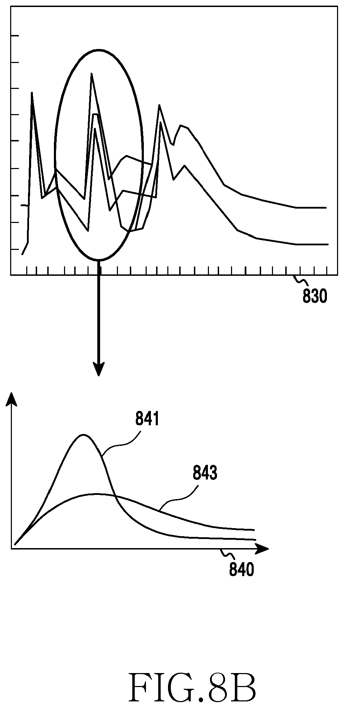

According to one example embodiment, as illustrated in FIG. 8A, the electronic device 101 may check a variation of user's blood sugar (e.g., a blood sugar graph 803 from FIG. 8A) based on biometric information data 801 that is a record of acquired biometric information. In case where the electronic device 101 checks a rise of the user's blood sugar based on the measured biometric information, the electronic device 101 may determine that a user takes food. For example, the electronic device 101 may distinguish food that the user takes based on a rising or falling degree (e.g., slope) of blood sugar. For example, referring to a blood sugar graph 840 of FIG. 8B, it may be checked that two or more curves 841 and 843 are generated at the same time point. The electronic device 101 may determine the type of carbohydrate that the user takes based on a slope of blood sugar variation, in a situation shown in the blood sugar graph 840.

Without being limited to determining the type of carbohydrate that the user takes as mentioned above, the electronic device 101 may determine the type of food (e.g., Korean food, Western food, Japanese food, etc.) that the user takes, and may also determine a situation such as whether the user has a meal or whether the user has tea. Further, without being limited to determining user's food intake or non-intake based on acquired biometric information, the electronic device 101 may determine user's various situations such as exercise, drinking, and working as well.

In operation 211, the electronic device 101 may determine the acquired position information as a position carrying out the determined specific situation. According to one example embodiment, the electronic device 101 may determine a meal situation corresponding to place information that is determined based on the user's situation information. The electronic device 101 may determine the checked place information as a meal place. According to one example embodiment, the electronic device 101 may store the determined place in the database. According to various example embodiments, in case where the position information of the electronic device 101 is determined as the meal place of the aforementioned description, the electronic device 101 may check the time of the electronic device 101 and check if the checked time is a mealtime stored in schedule information. In case where the time of the electronic device 101 corresponds to the mealtime, the electronic device 101 may determine that the user is in a meal situation. The electronic device 101 may perform an operation designated to the electronic device 101 correspondingly to the meal situation, such as, for example, an operation of determining that the user is in the meal situation, and measuring the user's blood sugar after the lapse of a designated time.

If performing operation 211, the electronic device 101 may end an example embodiment of FIG. 2.

In operation 213, the electronic device 101 may perform a designated operation based on the checked position information. According to one example embodiment, in case where it is determined that the checked position information is a meal place, the electronic device 101 may determine a user situation as a meal situation. According to one example embodiment, the electronic device 101 may determine that the user situation is the meal situation, and measure user's blood sugar after the lapse of a designated time. According to one example embodiment, in case where it is determined that there a sudden blood sugar variation indicating food intake does not occur as a result of measuring the user's blood sugar, the electronic device 101 may generate and store information indicating the erroneous determination that the user situation is the meal situation with respect to the position information of the electronic device 101.

If performing operation 213, the electronic device 101 may end the example embodiment of FIG. 2.

According to the aforementioned example embodiment, when describing a user situation corresponding to an operation, the electronic device 101 describes, though not limited to, for example, a lunch, but it is obvious that the present disclosure is applicable to position information of the electronic device 101 and various schedule information stored in the electronic device 101 or a behavior stored in a database. For example, the electronic device 101 may determine corresponding at least one situation among various situations such as exercise, driving, and traveling stored in the electronic device 101, based on position information and/or user's biometric information. Further, in case where the electronic device 101 determines one situation such as a meal state, the electronic device 101 may determine a sub category based on the user's biometric information checked in the electronic device 101. For example, in case where the electronic device 101 determines a user situation as a meal situation, the electronic device 101 may determine a menu (e.g., a carbohydrate-centered meal or a proteincentered meal) based on information of blood sugar variation or information of blood pressure variation, or may determine a detailed situation such as coffee drinking or nondrinking.

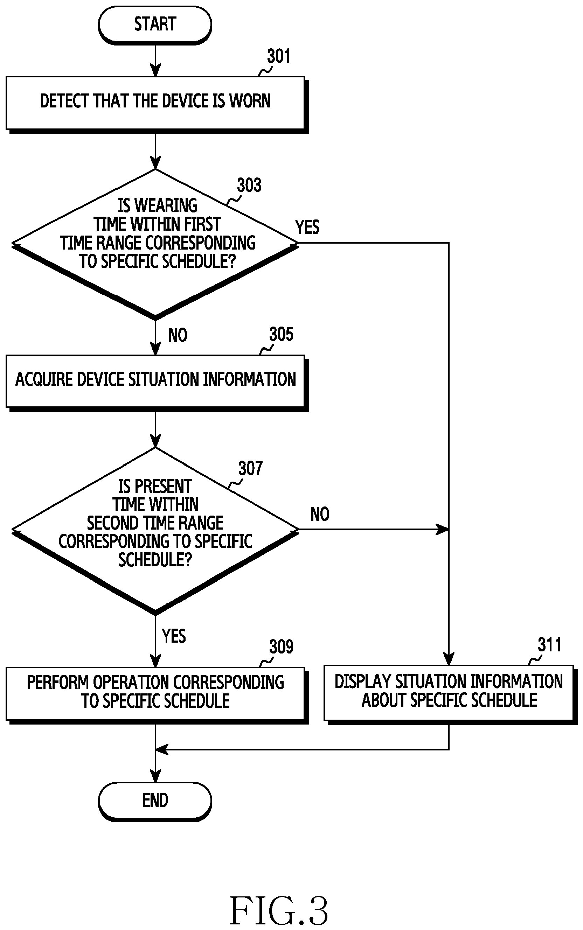

FIG. 3 illustrates a flow of an operation according to wearing in an electronic device according to various example embodiments.

In operation 301, the electronic device 101 may detect that the electronic device 101 is worn on a part of the body of a user. According to one example embodiment, the electronic device 101 may be a device of a wristwatch (or band) form, and may be a device worn on the wrist and acquiring user's biometric information. The electronic device 101 may check that the electronic device 101 is worn on the part of the body of the user, using at least one sensor such as a contact sensor of a buckle of the electronic device 101, a proximity sensor, or a temperature sensor.

In operation 303, the electronic device 101 may check whether a time at which the electronic device 101 is worn falls within a corresponding time range of a specific schedule. According to one example embodiment, the electronic device 101 may determine if the wearing time is within a first time range correspondingly to a specific schedule of the electronic device 101. For example, according to one example embodiment, in case where the electronic device 101 detects the wearing of the electronic device 101, the electronic device 101 may select a user's attendance schedule based on schedule information of the electronic device 101. In case where the electronic device 101 detects the wearing and selects the user's attendance schedule, the electronic device 101 may determine if the wearing time (e.g., present time at which the electronic device 101 is worn) of the electronic device 101 is included within a user's average wearing time range. For example, in case where a time at which the user wears the electronic device 101 is between 7:00 a.m. to 7:30 a.m. (e.g., within the first time range) based on the database, the electronic device 101 may determine if the wearing time of the electronic device 101 is within the first time range. In case where the wearing time is within the first time range, the electronic device 101 may perform operation 311. In case where the wearing time is not within the first time range, the electronic device 101 may perform operation 305.

In operation 305, the electronic device 101 may acquire device situation information. According to one example embodiment, the electronic device 101 may check the device situation information such as position information of a time point at which the electronic device 101 is worn on a part of the body of the user, weather information, etc. Further, the electronic device 101 may check information related with a specific schedule (e.g., attendance) such as traffic situation information through the Internet or server 106 coupled by network communication. In operation 307, based on the checked information, the electronic device 101 may determine if the time at which the electronic device 101 is worn on the part of the body of the user (e.g., a present time) is within a second time range. According to one example embodiment, based on the acquired information, the electronic device 101 may check a route capable of arriving up to a destination within the shortest time in relation with the specific schedule (e.g., attendance). The electronic device 101 may determine if the electronic device 101 can arrive at the destination within a time (e.g., an attendance time) designated to the specific schedule by using the shortest route. Here, the second time range may indicate a time range in which attendance is available within the attendance time (e.g., a time boundary determined to be lateness). In case where the time at which the electronic device 101 is worn on the part of the body of the user is within the second time range, the electronic device 101 may perform operation 309. In case where the time is out of the second time range, the electronic device 101 may perform operation 311.

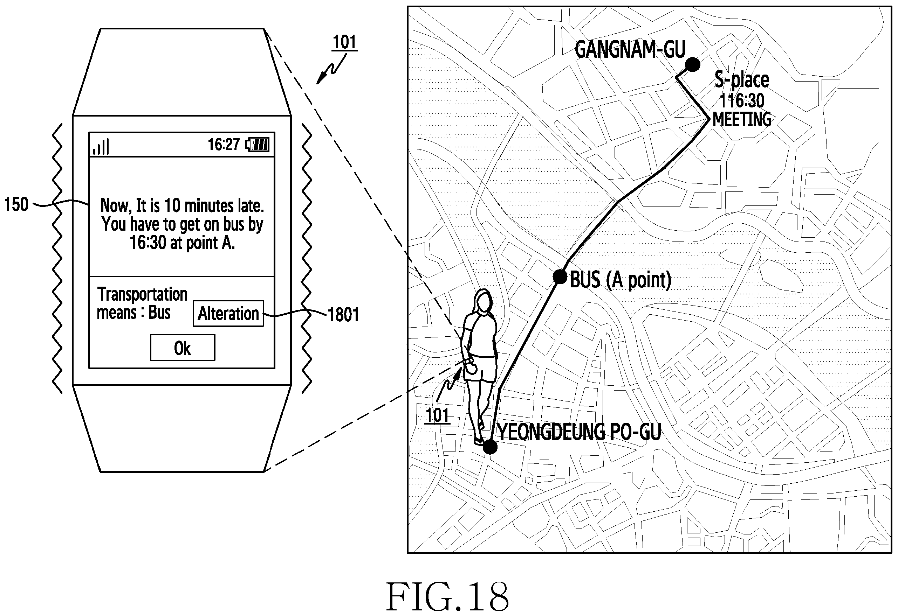

In operation 309, the electronic device 101 may perform a designated operation corresponding to the specific schedule (e.g., attendance) and the time at which the electronic device 101 is worn on the part of the body of the user. According to one example embodiment, the electronic device 101 may output, to the user, a shortest-time route capable of going to work without lateness. For example, as shown on the display 150 of FIG. 18, the electronic device 101 may display information that the user won't be late if arriving up to a specific position (e.g., a transfer section, point A) by a specific time. When displaying the corresponding information, the electronic device 101 may notify the user of a method of moving the shortest-time route, in consideration of a user's average movement speed or various movement methods such as using a transportation means (e.g., a taxi).

In operation 311, the electronic device 101 may perform a designated operation corresponding to preceding operation 303 or 307. According to one example embodiment, in case where the result of performing operation 303 is that the wearing time is within the first time range, the user can go to work without a concern about lateness, so the electronic device 101 may provide daily information to the user. For example, the electronic device 101 may provide information determined desirable for the user, such as a weather forecast, a temperature, a traffic situation, and a today's main schedule. Here, when determining the information determined desirable for the user, the electronic device 101 may determine, as the information determined desirable for the user, information requested to be provided at a designated count (e.g., three times) or more based on a user input at a corresponding time (or a corresponding time range).

According to various example embodiments, the electronic device 101 may display, on the display 150, information such as a time at which the user has to start to avoid lateness and a recommended transportation means. According to one example embodiment, the electronic device 101 may acquire traffic situation information through the Internet or another electronic device (e.g., server 106) coupled by network communication. For example, the electronic device 101 may acquire, as the traffic situation information, information such as road traffic situation information for the destination, a shortest-distance transportation means to the destination, a shortest-time transportation means to the destination, and an average required time for the destination, based on a designated designation corresponding to a schedule (e.g., attendance) checked at a time point at which the user wears the electronic device 101. The electronic device 101 may display, on the display 150, starting time (or recommended starting time) information for arriving without lateness by a time designated to a schedule (e.g., attendance), based on the acquired information.

If performing operation 311, the electronic device 101 may end an example embodiment of FIG. 3.

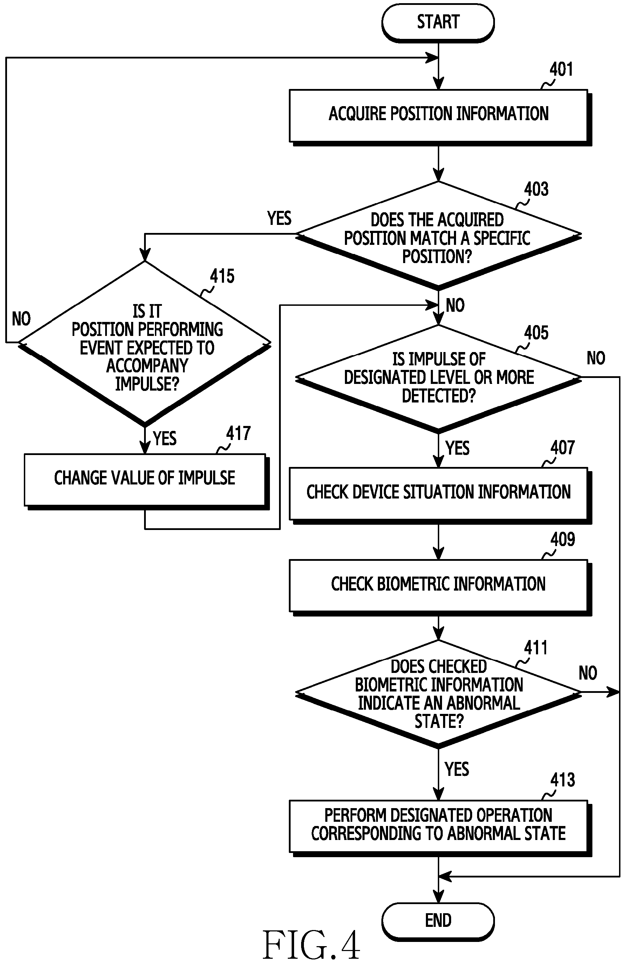

FIG. 4 illustrates a flow of an operation according to an exceptional situation in an electronic device according to various example embodiments.

Referring to operation 401, the electronic device 101 may acquire position information of the electronic device 101 through the sensor unit 170. According to one example embodiment, when the electronic device 101 acquires the position information of the electronic device 101 through the sensor unit 170, the electronic device 101 may acquire the position information at a designated time period, in real-time, or at a designated time range.

Referring to operation 403, the electronic device 101 may determine if a position of the electronic device 101 is a specific event (e.g., a specific position). According to one example embodiment, in case where the electronic device 101 acquires the position information of the electronic device 101 in real-time, the electronic device 101 may check if the position of the electronic device 101 is the specific position designated to a database (e.g., a playground). In case where the positon information acquired in the electronic device 101 is the specific position designated to the database, the electronic device 101 may perform operation 415 and, otherwise, the electronic device 101 may perform operation 405.

Referring to operation 415, the electronic device 101 may determine if the specific position determined based on the position information is a position in which impulse of a user's movement is expected. An "impulse" may be used herein refer to a start or initiation of some user movement, whether controlled (e.g., a particular physical exercise movement) or uncontrolled (e.g., falling from a height, being pushed by another person).

According to one example embodiment, in case where it is determined that the position of the electronic device 101 is the specific position (e.g., the playground) expected to accompany the impulse of the user's movement, the electronic device 101 may proceed to operation 417 and, otherwise, may repeatedly perform operation 401.

In operation 417, the electronic device 101 may change a reference value (e.g., a threshold value) of the impulse applied to the electronic device 101 correspondingly to the specific position, based on a database.

According to one example embodiment, in case that it is determined that the position of the electronic device 101 is the position in which the user's movement is accompanied by the impulse, the electronic device 101 may change the reference value of the impulse. For example, in case where it is determined that the electronic device 101 is located in a football field, the electronic device 101 may detect a designated impulse corresponding to running from the database, and set the detected impulse as the reference value of the impulse. According to various example embodiments, in case where it is determined that the electronic device 101 is located in a boxing ring, the electronic device 101 may detect a designated impulse corresponding to a punch from the database, and set the detected impulse as the reference value of the impulse.

Referring to operation 405, the electronic device 101 may determine if the impulse acquired through the sensor unit 170 exceeds the reference value of the impulse. According to one example embodiment, for example, in case where it is determined that the position of the electronic device 101 is not the specific position designated to the database, the electronic device 101 may check a first reference value that is set to setting information, and set the checked first reference value as the reference value of the impulse. According to one example embodiment, the electronic device 101 may acquire an impulse applied to the electronic device 101 based on a user movement, and may check if it detects an impulse of the first reference value or more.

According to various example embodiments, in case where the reference value of the impulse is changed through operation 415 and/or operation 417, the electronic device 101 may check if an impulse exceeding the changed reference value is detected. For example, the electronic device 101 may determine the position of the electronic device 101 as the boxing ring designated to the database. The electronic device 101 may detect a second reference value corresponding to an impulse designated to the boxing ring, based on the database, and set the detected second reference value as the reference value of the impulse. The electronic device 101 may check if the impulse detected through the sensor unit 170 exceeds the second reference value.

In case where the detected impulse exceeds a first impulse or a second impulse according to the position information of the electronic device 101, the electronic device 101 may perform operation 407 and, otherwise, the electronic device 101may end an example embodiment of FIG. 4.

Referring to operation 407, the electronic device 101 may check device situation information through the sensor unit 170. According to one example embodiment, the electronic device 101 may predict a situation in which a user is at risk or will be injured by a impulse, based on information such as an acceleration of the electronic device 101, a movement direction thereof, and an impulse thereof through the sensor unit 170. For example, in case where the electronic device 101 detects an acceleration of gravity direction, a movement of a designated distance or more of the electronic device 101 and an impulse exceeding a reference value, the electronic device 101 may predict that it is a situation in which the user is falling. For example, in case where the first impulse exceeding the first reference value (or second reference value) and the second impulse exceeding a third reference value are detected in a horizontal direction, the electronic device 101 may predict that the user has collapsed due to a shock. Further, after the acceleration of gravity direction and the impulse exceeding the designated reference value are detected, in case where a movement of the electronic device 101 is not detected for a designated time, the electronic device 101 may determine that the user has fainted.

As described above, the electronic device 101 may predict user situation information by combining sensor values measured for a designated movement of the electronic device 101 based on the database.

According to one example embodiment, without determining the user situation information by a combination of the device situation information described through operation 407, the electronic device 101 may further include biometric information in the device situation information and determine the user situation information as in operation 409 described later.

Referring to operation 409, the electronic device 101 may acquire biometric information of a user who wears the electronic device 101, based on at least one medical sensor included in the sensor unit 170. According to one example embodiment, the electronic device 101 may acquire the biometric information such as blood sugar of the user who wears the electronic device 101, a body composition, a body temperature, a pulse, a heart rate, and a blood pressure through the medical sensor. The electronic device 101 may predict a situation of the user who wears the electronic device 101, based on the acquired biometric information and/or the database. For example, the electronic device 101 may determine if heavy bleeding occurs from the user based on the acquired blood pressure.

According to various example embodiments, without determining the user situation based on the biometric information, the electronic device 101 may determine the user situation using sensor values (e.g., device situation information) measured for a designated movement of the electronic device 101 and the biometric information.

Referring to operation 411, the electronic device 101 may determine if the user situation (or user's state) determined based on the device situation information and/or the user's biometric information checked through operation 407 and/or operation 409 is an abnormal situation.

According to one example embodiment, in case where it is determined that the user situation is a falling and/or fainting situation based on the device situation information, the electronic device 101 may check the user's biometric information. The electronic device 101 may check a user's blood sugar graph (e.g., the blood sugar graph 803 of FIG. 8A (and/or the graph 840 of FIG. 8B) and check that user's blood sugar is below a designated level, and may determine that it is a situation in which the user has collapsed due to a shock induced by low blood sugar.

According to one example embodiment, in case that it is determined that the user suffers an impulse exceeding a designated reference value based on the device situation information, the electronic device 101 may check the user's biometric information. The electronic device 101 may check a user's blood pressure and check that the blood pressure is below a designated level, and may determine that bleeding occurs due to the impulse that the user suffers.

According to one example embodiment, in case where it is checked that the user is in an abnormal situation, the electronic device 101 may perform operation 413 and, otherwise, the electronic device 101 may end the example embodiment of FIG. 4.

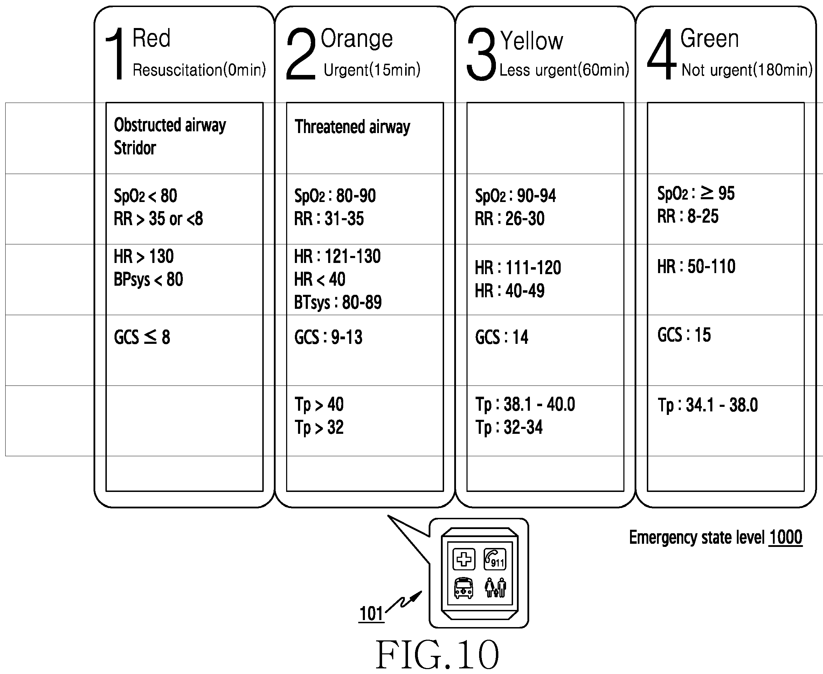

Referring to operation 413, the electronic device 101 may perform a designated operation corresponding to the checked user situation. According to one example embodiment, as in FIG. 10, the electronic device 101 may divide the user's abnormal situation into two or more steps (or levels) and store in the database. The electronic device 101 may compare the device situation information detected through the sensor unit 170 and the user's biometric information with the database, and determine the user's abnormal situation step. For example, the electronic device 101 may determine a user's abnormal state level with reference to the database based on the user's biometric information. The electronic device 101 may determine a user's abnormal state such as user's fainting, shock, and bleeding based on the biometric information such as detected blood sugar, body composition, body temperature, pulse, heart rate, and blood pressure.

According to various example embodiments, the electronic device 101 may perform a designated operation corresponding to the user's abnormal situation step that is determined based on the user situation information. For example, the designated operation may be implemented as a table and included in the database like an emergency state level 1000 illustrated in FIG. 10. For example, without being limited to being included in the database in a table form as mentioned above, data such as the emergency state level 1000 may be included in the database in various forms such as a data table and a data sheet.

According to one example embodiment, in case where it is determined that the user's abnormal situation step is a step 4 (green), the electronic device 101 may determine that the user situation is a normal situation. According to one example embodiment, in case where it is determined that the user's abnormal situation step is a step 3 (yellow), the electronic device 101 may perform a designated operation in which a user can realize his/her own state. For example, the electronic device 101 may output a vibration using at least one motor. For another example, if it is determined that the user's abnormal situation is a shock caused by low blood sugar, the electronic device 101 may output a notification of notifying a need for sugar intake. For example, the electronic device 101 may display the notification on the display 150 or output the notification by an audio through a speaker.

According to one example embodiment, if it is determined that the user's abnormal situation step is a step 2 (orange), the electronic device 101 may send a call and/or transmit a designated message to a contact (e.g., a family) designated to an emergency calling number. For example, if it is determined that the user situation is a fainting state, the electronic device 101 may perform an electric shock for correcting a user's posture or awakening a user's consciousness, for a user.

According to one example embodiment, if it is determined that the user's abnormal situation step is a step 1 (red), the electronic device 101 may send a call and/or transmit a designated message to a contact (e.g., 119 of South Korea or 911 of USA) designated to a state-designated emergency calling number. In case where it is determined that the user's state is a situation requiring emergency relief activities, the electronic device 101 may perform a designated operation. For example, in case where it is determined that the user's fainting situation is caused by a shock of low blood sugar, and is an emergency (e.g., a blood sugar level is equal to or is less than a designated level) based on biometric information, the electronic device 101 may give insulin shot to a user.

According to one example embodiment, in case where it is determined that the user situation is a fainting situation and a non-breathing situation, the electronic device 101 may operate a defibrillator. For example, the electronic device 101 may be in a state of being wiredly or wirelessly coupled with the defibrillator located on the chest of a user. The electronic device 101 may acquire biometric information in real-time correspondingly to the user situation, and may operate the defibrillator based on the biometric information.

After operation 413, the electronic device 101 may end the example embodiment of FIG. 4.

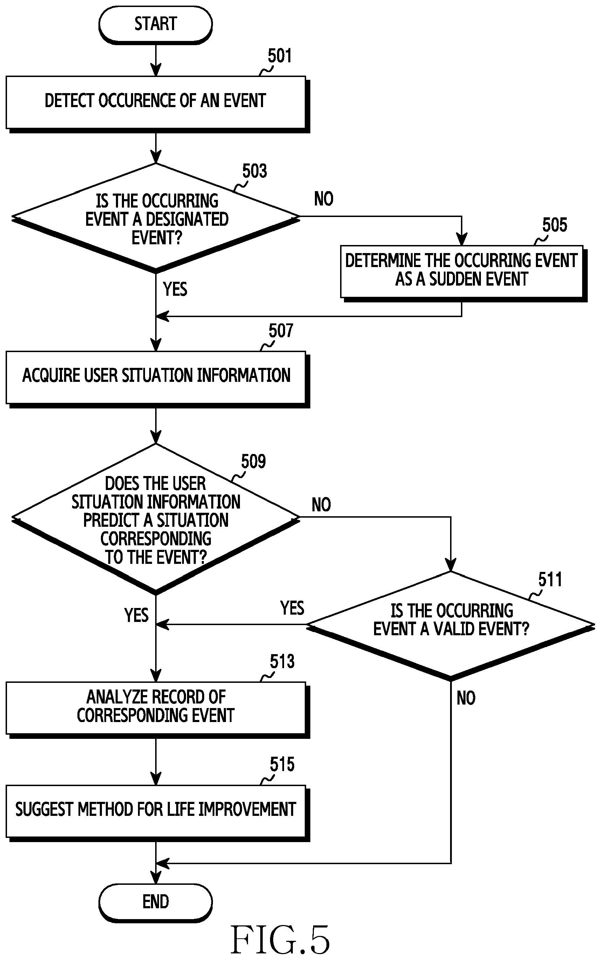

FIG. 5 illustrates a flow of an operation of utilizing acquired information in case of a valid event in an electronic device according to various example embodiments.

In operation 501, the electronic device 101 may detect the occurrence of an event. According to one example embodiment, the electronic device 101 may determine the occurrence or non-occurrence of the event, based on device situation information acquired through the sensor unit 170. For example, the electronic device 101 may check the occurrence or non-occurrence of a specific event, based on device situation information such as position information of the electronic device 101, a time, an impulse and/or the wearing of the electronic device 101.

Referring to operation 503, the electronic device 101 may determine if the occurring event is an event designated by a schedule. According to one example embodiment, the electronic device 101 may include at least one event in the schedule information based on a user input, or predicted via algorithmic learning (e.g., an event occurring based on an operation repeatedly carried out at a designated count or more) of the electronic device 101. In case where the event occurs based on device situation information, the electronic device 101 may determine if the corresponding event is an event existing within the schedule information.

According to one example embodiment, in case where the electronic device 101 stops at a specific position for a designated time (e.g., five minutes) or more, the electronic device 101 may determine if the specific position is a designated position corresponding to a designated event based on schedule information.

According to one example embodiment, in case where the specific position of the electronic device 101 is a position of a specific event stored in schedule information, the electronic device 101 may check if a time of arrival to the specific position is within a time range of the specific event stored in the schedule information.

According to one example embodiment, in case where the electronic device 101 detects user's wearing of the electronic device 101 (e.g., detects a combination of a buckle of the electronic device 101 in case where the electronic device 101 is of a wristwatch form), the electronic device 101 may check if the wearing of the electronic device 101 and/or the wearing time of the electronic device 101 corresponds to a specific event stored in schedule information. Without being limited to the aforementioned method, the electronic device 101 may check if a corresponding event exists within the schedule information based on device situation information and schedule information.

According to various example embodiments, without being limited to checking if an event corresponding to device situation information is a designated event based on schedule information, the electronic device 101 may check if the event corresponding to the device situation information is the designated event based on a database stored in the memory 130 of the electronic device 101. According to one example embodiment, the database of the electronic device 101 may store a specified at least one event that is set based on the device situation information of the electronic device 101 and/or biometric information of a user. For example, in case where the same or similar operation that is detected based on the device situation information of the electronic device 101 and/or the biometric information of the user is repeatedly performed at a designated count or more, the electronic device 101 may store, in the database, the same or similar operation as an event such as user's habits or tastes.

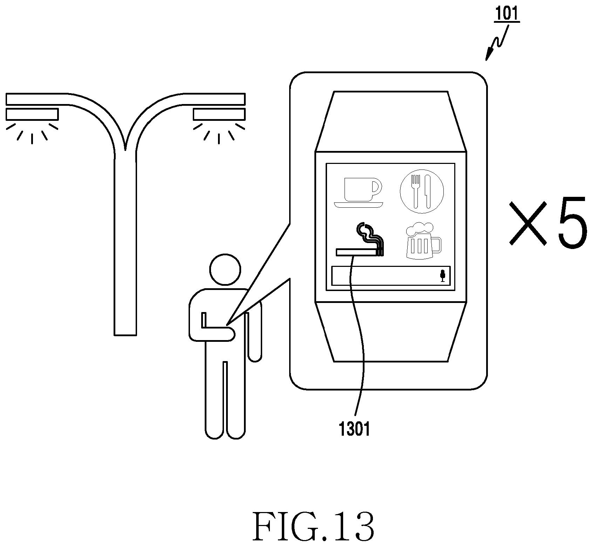

According to one example embodiment, if user's smoking is checked at a designated count (e.g., five times) or more in a specific position (or a specific range, such as within a three-meter radius of the specific position), the electronic device 101 may designate the user's smoking in the corresponding position as a user's habit event and store the habit event in the database. According to one example embodiment, in case where the habit event occurs, the electronic device 101 may display, on the display 150, a notification 1301 of notifying the user's smoking as shown in FIG. 13.

According to one example embodiment, in case where the occurring event is an event not designated to schedule information, the electronic device 101 may check if the occurring event is an event designated to a database. For example, in case where the occurring event is a user's smoking event, the electronic device 101 may check if the smoking event is the event not designated to the schedule information but is the event designated to the database.

According to one example embodiment, in case where the occurring event is a designated event, the electronic device 101 may proceed to operation 507 and, otherwise, the electronic device 101 may proceed to operation 505.

In operation 505, the electronic device 101 may determine the occurring event as a sudden event. For example, the sudden event may represent an event designated neither to the schedule information nor to the database.

Referring to operation 507, the electronic device 101 may acquire user situation information. For example, the user situation information may include user's biometric information that is detected through at least one medical sensor included in the sensor unit 170. And, the electronic device 101 may check a user situation (e.g., fainting, bleeding, shock, smoking, etc.) using the detected biometric information. For example, the electronic device 101 may determine the user situation corresponding to the user's biometric information based on at least one user situation table (or sheet) included in the database.

Referring to operation 509, the electronic device 101 may determine if the determined user situation is a user situation corresponding to the designated event.

According to one example embodiment, in case where the occurring event is a user's meal schedule, the electronic device 101 may determine if a user had a meal based on user situation information. For example, the electronic device 101 may determine if a position of the electronic device 101 is a position designated as a meal place through operation 501 and/or operation 503. Further, the electronic device 101 may check if the user had the meal based on biometric information. According to one example embodiment, in case where it is checked that the user had the meal, the electronic device 101 may determine that the determined user situation is a predicted situation corresponding to the designated event and, otherwise, the electronic device 101 may determine that the determined user situation is not the predicted situation corresponding to the designated event.

According to one example embodiment, in case where the determined user situation is smoking, the electronic device 101 may determine that the determined user situation is the predicted situation corresponding to the designated event and, otherwise, the electronic device 101 may determine that the determined user situation is not the predicted situation corresponding to the designated event.

According to one example embodiment, in case where it is determined that the determined user situation is the predicted situation, the electronic device 101 may perform operation 513 and, otherwise, the electronic device 101 may perform operation 511.

Referring to operation 511, the electronic device 101 may check if the occurring event (e.g., sudden event) is a valid event. According to one example embodiment, in case where a position of the electronic device 101 is not a specific place (e.g., at least one place among positions designated as meal places to a database or schedule information) of a designated event, the electronic device 101 may output (e.g., display on the display 150) a notification of notifying that a user's position is not one place among the positions designated to the schedule information (or the database).

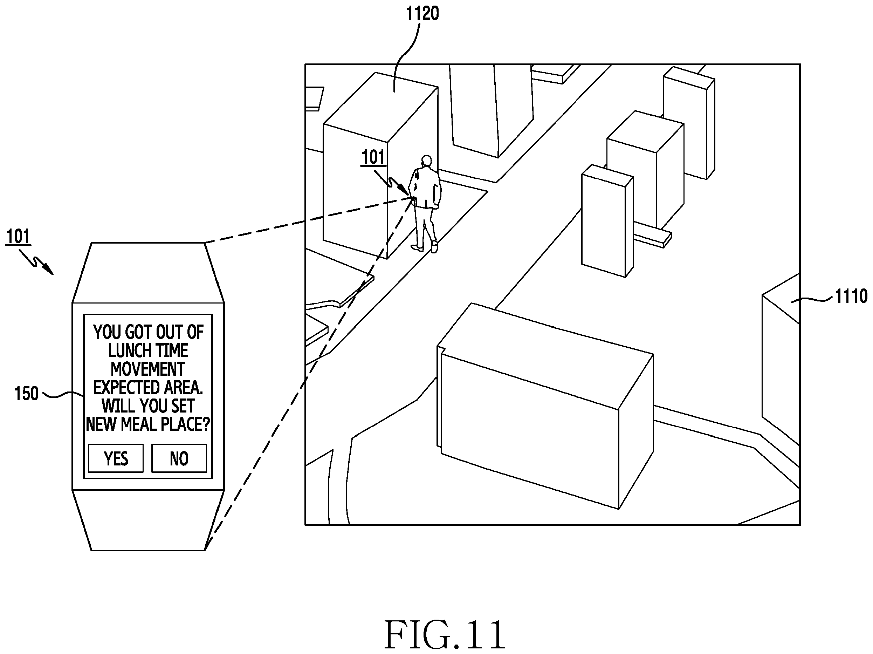

According to one example embodiment, in case where an occurring time of the occurring event is a mealtime and a user's positon (e.g., a position 1120) is not a position designated to the specific restaurant 1110 of FIG. 11, the electronic device 101 may display, on the display 150, a notification of getting out of the meal place and inquiring whether to set the current user's position as a new meal place. According to one example embodiment, the electronic device 101 may determine the position 1120 as the new meal place based on a user input, and may determine the occurring event as the valid event.

If the checking result is that the occurring event is the valid event, the electronic device 101 may proceed to operation 513 and, otherwise, the electronic device 101 may end an example embodiment of the FIG. 5.

Referring to operation 513, the electronic device 101 may analyze a record (e.g., log data) of the corresponding event. According to one example embodiment, in case where the occurring event is a meal schedule, the electronic device 101 may analyze information about the corresponding event and the last meal schedule of at least once or more. For example, the electronic device 101 may analyze information about a meal that a user has in the corresponding meal schedule and information about a meal that the user had in the last meal schedule of at least once or more and/or meal information such as a meal interval, based on biometric information such as blood sugar and information recorded in relation with the meal. According to one example embodiment, the electronic device 101 may acquire information about a user's current meal habit based on the analysis information.

According to one example embodiment, in case where the occurring event is a smoking event, the electronic device 101 may analyze information about the corresponding smoking event and the last smoking event of at least once or more. For example, the electronic device 101 may analyze smoking information such as the number of cigarettes smoked in the corresponding smoking event and the number of cigarettes smoked in the last smoking event and/or a smoking interval, based on biometric information such as blood sugar and information recorded in relation with smoking. According to one example embodiment, the electronic device 101 may acquire information about a user's smoking habit, based on the analysis information.

Referring to operation 515, the electronic device 101 may suggest a method for improvement of a user's life based on the acquired information. According to one example embodiment, in case where the occurring event is a habit event, the electronic device 101 may compare information recorded in relation with a user's habit and the corresponding event with a designated target value and provide a next event execution condition. For example, in case where carbohydrate intake detected based on blood sugar analysis information is equal to or is greater than a reference value (e.g., a daily average intake of an adult), the electronic device 101 may check a target value (e.g., the carbohydrate content of food that is set or designated by a user). The electronic device 101 may provide a meal menu of less carbohydrate content as a recommended menu of a next meal, based on the checked target value.

According to one example embodiment, when providing a recommended menu of a next meal of less carbohydrate content, the electronic device 101 may also provide the recommended menu to make the carbohydrate content equal to a target value over a designated count (e.g., ten times), without providing a recommended menu including food whose carbohydrate content sharply falls compared to current food. According to one example embodiment, when providing the recommended menu to reduce carbohydrate content to a target value over twice or more, the electronic device 101 may reflect analysis information about intake food and determine a next provided recommended menu. According to one example embodiment, the electronic device 101 may utilize analysis information corresponding to various living activities such as eating, smoking, drinking, and exercise and provide a condition of life improvement.

If performing operation 515, the electronic device 101 may end an example embodiment of FIG. 5.

FIG. 6 is a diagram related with an event stored in an electronic device according to various example embodiments.

Based on a generated input, the electronic device 101 may predict a user's behavior and/or a user situation with reference to a database. Here, the generated input may be device situation information and/or biometric information detected through the sensor unit 170 of the electronic device 101. In case where it is checked that the generated input is an event designated to schedule information and/or the database, the electronic device 101 may determine that the user's behavior is a daily situation 600.

According to various example embodiments, even if the generated input is not the event designated to the schedule information and/or the database, the electronic device 101 may generate an event in the database based on a user's behavior repeatedly carried out at a designated count or more. In case where the input corresponding to the generated event is generated, the electronic device 101 may determine that the user's behavior is the daily situation 600.

Further, in case where a designated event repeatedly occurs in a state in which the electronic device 101 is not worn by a user, the electronic device 101 may generate the corresponding event in the database. According to one example embodiment, the electronic device 101 may check that a situation in which the electronic device 101 is not worn is repeated at a designated count or more during a time range including a time (e.g., 24:00 to 03:00) designated to night. For example, the electronic device 101 may check an event in which the electronic device 101 is not worn during a time range of 23:00 to 06:00 and, in case where the corresponding event occurs at a designated count (e.g., 10 times) or more, the electronic device 101 may determine the corresponding time range, as the daily situation 600 corresponding to a user's sleep time.

FIG. 7 is a diagram related with an event not stored in an electronic device according to various example embodiments.

According to various example embodiments, a user who wears the electronic device 101 may check various user situations through the sensor unit 170 besides an event stored in schedule information of the electronic device 101.

Referring to Case 2, in case where the electronic device 101 detects that a user is consuming beverages in a location not stored within schedule information and a database, and at a time at which no schedule is stored through the sensor unit 170, the electronic device 101 may determine it as an exceptional situation 700, such as when the user spontaneously meets a friend.

Referring to Case 3, in case where the electronic device 101 detects an impulse exceeding a designated reference value through the sensor unit 170, or where the electronic device 101 detects a position movement having a designated acceleration and/or designated height or more in a particular direction indicated by the force of gravity, the electronic device 101 may determine that an exceptional situation 700 is occurring, corresponding to an injury to the user.

Referring to Case 4, in case where the electronic device 101 detects that the user is not moving during a designated time through the sensor unit 170, and/or the electronic device 101 detects that the user is not breathing during a designated time, the electronic device 101 may determine it as an exceptional situation 700 corresponding to the user fainting and/or being in shock. For example, in a method of detecting that the user wearing the electronic device 101 is not moving during a designated time, the electronic device 101 may detect that there is no acceleration variation of a specific value or more during a designated time (e.g., 10 seconds) or more through the sensor unit 170 (e.g., an acceleration sensor), the electronic device 101 may thereby determine that the user is not moving.

Thus, the electronic device 101 may measure a variety of exceptional situations 700. With reference to information detected through the sensor unit 170 of the electronic device 101 and/or the database, the electronic device 101 may determine various exceptional situations, besides the aforementioned various exceptional situations described above.

FIG. 8A is a diagram illustrating an operation of determining a user situation dependent on blood sugar variation in an electronic device according to various example embodiments.

According to various example embodiments, the electronic device 101 may detect biometric information of a user through the sensor unit 170, and may generate biometric information data 801 based on the biometric information detected at least once or more. When the electronic device 101 detects the biometric information of the user, the electronic device 101 may detect the biometric information such as blood sugar, a body composition, a body temperature, a pulse, a heart rate, an EDA, a GSR, and a BIA. When the electronic device 101 detects the biometric information of the user, the electronic device 101 may detect the biometric information at a designated time interval, and may detect the biometric information in accordance with designated device situation information of the electronic device 101, or may detect the biometric information in real-time. The electronic device 101 may determine a user situation based on the detected biometric information. According to one example embodiment, the electronic device 101 may check a user's blood sugar graph 803 based on blood sugar information received via the biometric information data 801. With reference to the blood sugar graph 803, the electronic device 101 may check information about a time point at which the user takes food, a time interval at which the user takes food, the type of the taken food and/or a blood sugar variation.

According to various example embodiments, without being limited to outputting information about food that the user takes at a time point of detecting the biometric information, when the electronic device 101 selects a specific time point 810 in the blood sugar graph 803 based on a user input, the electronic device 101 may display, on the display 150, blood sugar information 811 corresponding to the selected time point 810.

According to various example embodiments, when the electronic device 101 detects a blood sugar level, the blood sugar level is frequently varied depending on a meal amount, a meal type, and a body activity, etc. and therefore, to detect a blood sugar level for utilizing as a health index, the electronic device 101 may change a blood sugar measurement time point by setting the measurement time point and frequency based on a user's behavior, without being limited to a user input and a periodical measurement. Here, the blood sugar measurement time point may mainly classified into three time points (i.e., empty stomach, two hours after a meal, and before going to bed), and may have a different criterion in determining a stability state in accordance with each case.

According to various example embodiments, the unit of a blood sugar level may be divided into the Conventional Unit scheme (mg/dL) used in Republic of Korea, or the SI Unit scheme (mmol/L) used in European zone. The mg/dL may be determined as a capacity of glucose included per blood 100 cc, and may be converted into 1 mmol/L = 18 mg/dL.

According to various example embodiments, when determining a blood sugar measurement frequency and time point, the electronic device 101 may include an algorithm determining automatically based on biometric information acquisition such as user's movement tracking, and may provide a function such as meal environment analysis (i.e., meal amount, meal type, and mealtime analysis) through blood sugar tracking and user notification (i.e., danger notification, medication time notification, a meal pattern, a meal balance, meal menu recommendation, and required-momentum notification) based on this.

According to various example embodiments, without being limited to providing a record (e.g., log data) of blood sugar information as described above, the electronic device 101 may provide a record of user's various biometric information.

According to various example embodiments, the electronic device 101 may detect a biometric information variation such as user's food intake or exercise, and may perform a specific function (e.g., a specific program such as a health care program) designated to the electronic device 101 based on the detected biometric information variation.

FIG. 8B is a diagram illustrating an operation of analyzing detected biometric information in an electronic device according to various example embodiments.

According to various example embodiments, the electronic device 101 may analyze user's biometric information detected through the sensor unit 170. According to one example embodiment, referring to a blood sugar graph 830, in case where a user has a meal, the electronic device 101 may determine the kind of the meal. For example, the electronic device 101 may predict the type of carbohydrate that the user takes based on a Glycemic Index (GI) that is expressed through blood sugar variation curves of the blood sugar graph 830. According to one example embodiment, referring to a partial graph portion indicated by graph 840 determining that the user has a meal in the blood sugar graph 830, the electronic device 101 may identify a blood sugar variation curve 841 indicating a high glycemic index via a sharp rise and fall of the glycemic index, a blood sugar variation curve 843 indicating a low glycemic index via a moderate rise and fall of the glycemic index, and a blood sugar variation curve of a middle glycemic index. Here, in case of contained carbohydrate showing the high glycemic index, it may be food such as a carrot, a honey, a corn flake, a wheat flour, a white boiled rice, a potato, a white bread, a banana, and a raisin. In case of carbohydrate showing the middle glycemic index, it may be food such as a corn, an oat mill, an orange, a white pasta, a sweet potato, a whole wheat pasta, and a pea. In case of carbohydrate showing the low glycemic index, it may be food such as a kidney bean, a lentil, an apple, a pearl, a cucumber, a tomato, a peanut, a walnut, a pine nut, and a broccoli. The electronic device 101 may check the type of carbohydrate that the user takes during a meal, based on slopes of the blood sugar variation curves.

According to various example embodiments, the electronic device 101 may check information about user's eating habits based on the blood sugar graph 830. The electronic device 101 may check user's health information based on biometric information, and provide a desirable meal menu to a user with reference to the checked eating habits.

According to various example embodiments, the electronic device 101 may check a time point at which a user has a meal based on the blood sugar variation curves of the blood sugar graph 830 and/or a blood sugar level of detected biometric information. In case where the user takes medicine, the electronic device 101 may check a medication time point based on the biometric information. In case where the user takes medicine related with a meal time point, if detecting a time point at which the user starts having a meal based on a blood sugar variation, the electronic device 101 may determine a medication time, and output a notification to the user at a medication time point.

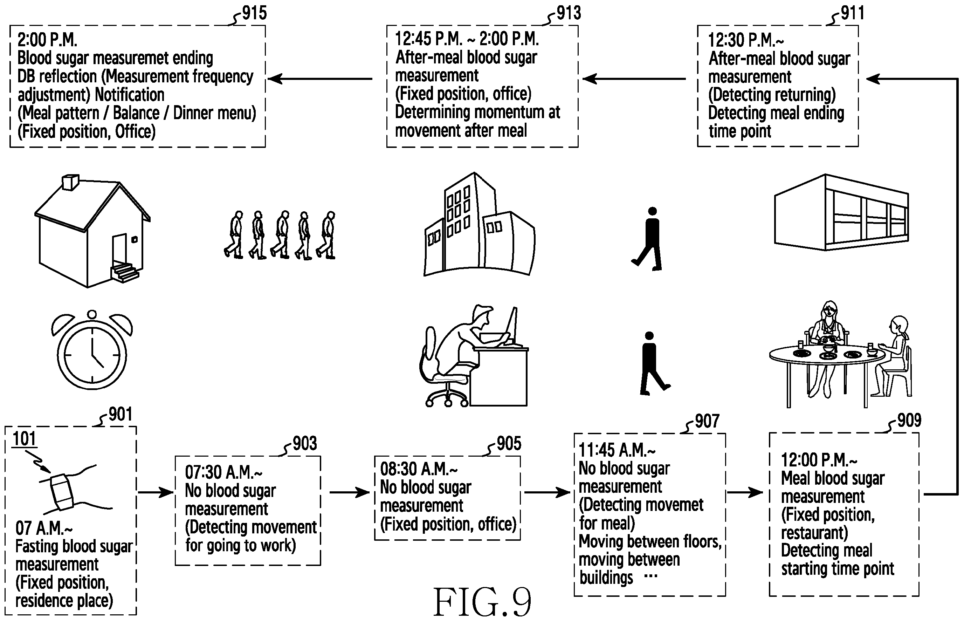

FIG. 9 is a diagram illustrating an operation of determining a user's state based on detected biometric information in an electronic device according to various example embodiments.

The electronic device 101 may acquire biometric information of a user, based on position information, time information and/or setting information. According to one example embodiment, in case where the electronic device 101 detects that the electronic device 101 is worn on a part of the body of the user, the electronic device 101 may determine a time point of acquiring the biometric information of the user. For example, the time point of acquiring the biometric information of the user may be a previously designated time interval. For another example, the time point of acquiring the biometric information of the user may be when the electronic device 101 is located in a designated place, when a set time arrives and/or when the electronic device 101 checks that specific biometric information of the user is changed.

Referring to FIG. 9, in case where a biometric time point reaches a first time point 901 and the electronic device 101 is worn on a part of the body of a user, the electronic device 101 may measure user's blood sugar using a sensor, and generate blood sugar information including a level of the measured blood sugar. Or, the electronic device 101 may check the user's blood sugar information from biometric information that is measured at the biometric time point (e.g., the first time point 901) as well.

And, the electronic device 101 may determine if the user has a meal based on the blood sugar information generated at the first time point 901. If it is determined that the user has the meal, the electronic device 101 may check information such as the type of food that the user takes based on the blood sugar information. If it is determined that the user does not have the meal, when generating the blood sugar information, the electronic device 101 may store the blood sugar information together with a blood sugar level of an empty stomach state. Here, as in FIG. 9, the first time point 901 may be a previously designated time, for example, 07:00, but the present disclosure is not limited to this. For example, the electronic device 101 may set, as the first time point 901, a time point at which the electronic device 101 is worn on the body of the user.

In case where the biometric time point reaches a second time point 909, the electronic device 101 may generate user's blood sugar information, and check if the user starts having a meal at the second time point 909 based on the generated blood sugar information. For example, the electronic device 101 may compare the blood sugar information generated at the second time point 909 and the blood sugar information generated at the first time point 901, thereby checking user's meal starting or non-starting.

Or, in case where the biometric time point reaches the second time point 909, the electronic device 101 may check the user's meal starting or non-starting based on a user's behavior (or movement) pattern and/or periodically acquired user's biometric information. According to one example embodiment, the user's behavior pattern may be determined based on information such as a position movement of the electronic device 101, a time, a movement speed, and a height change. For example, the electronic device 101 may determine a user's behavior such as an attendance time point 903, work 905, and a movement 907 for meal, based on the user's behavior pattern.

For another example, the electronic device 101 may determine the user's meal starting or non-starting based on a variation of information such as a body temperature, blood sugar, a perspiration composition, and a body composition which are included in acquired biometric information. If it is determined that the user starts a meal, the electronic device 101 may generate user's blood sugar information, and directly check if the user starts the meal at the second time point 909 based on the generated blood sugar information.

For further example, the electronic device 101 may determine the user's meal starting or non-starting in accordance with the user's behavior (or movement) pattern (for example, in accordance with whether a current position of the electronic device 101 is the same as a previously stored position of at least one restaurant). If the user's behavior pattern is the same as the previously stored user's behavior pattern, the electronic device 101 may determine that the user starts the meal.

In case where it is determined that the user starts the meal, the electronic device 101 may actually detect the meal starting or non-starting by comparing user's blood sugar at a previously designated time interval. For example, the electronic device 101 may measure a blood sugar level at a two-minute interval during ten minutes, and determine if the user starts the meal based on a variation of the measured blood sugar level. If it is determined that the user starts the meal, the electronic device 101 may generate user's blood sugar information and store the generated blood sugar information.

For another example, the electronic device 101 may determine user's meal or non-meal in consideration of all a user's behavior pattern and periodically acquired user's biometric information.

In case where the biometric time point reaches a third time point 911, the electronic device 101 may check user's blood sugar information based on acquired biometric information. According to one example embodiment, in case where the third time point 911 is determined to be a time when a designated time (e.g., thirty minutes) elapses from a time point (e.g., the second time point 909) at which the user starts a meal, the electronic device 101 may measure user's blood sugar using the sensor, and generate blood sugar information including a level of the measured blood sugar. The electronic device 101 may store information such as meal ending, a current time, and the type of taken food, together with the generated blood sugar information.

In case where the biometric time point reaches a fourth time point 913, the electronic device 101 may check user's blood sugar information and/or user's activity information. According to one example embodiment, the electronic device 101 may check user's biometric information during a previously designated time range from the time point (e.g., third time point 911) at which the user ends a meal, and check information such as a momentum of a user, a body composition variation, and a perspiration composition variation. The electronic device 101 may check if the user continuously moves based on the biometric information. If it is determined that the user continuously moves (e.g., a state in which movement is continued for ten minutes or more), the electronic device 101 may determine, as a biometric range of the fourth time point 913, from the third time point 911, a movement starting time point, to a movement ending time point. The electronic device 101 may acquire biometric information of many times at the fourth time point 913, and may measure blood sugar of at least once or more when acquiring each biometric information. Further, the electronic device 101 may acquire information such as a moved distance, a calorie expenditure, a capacity of perspiration, a perspiration composition variation, and a body composition, and may store the acquired information together with blood sugar information.

In case where the biometric time point reaches a fifth time point 915, the electronic device 101 may end blood sugar measurement corresponding to one period. Here, the one period may be a time range corresponding to one day. For example, the electronic device 101 may check a starting time of one period and an ending time thereof, based on setting information. Referring to an example embodiment of FIG. 9, the starting time of one period being set to the electronic device 101 may be determined to be 07 a.m., and the ending time may be determined to be 02 p.m.

In case where the electronic device 101 checks a designated time (e.g., 2 p.m.), the electronic device 101 may determine, as information of one period, biometric information, blood sugar information, momentum information, perspiration composition information, and body composition information that are generated from the first time point 901 to the latest time point (e.g., the fifth time point 915), and information stored together with such the information. The electronic device 101 may determine information such as a recommended menu of a next meal, a recommended exercise intensity, and a recommended consumption calorie, based on the information of one period.

According to another example embodiment, in case where biometric information acquired after a time point of checking the ending of one period is one acquired at the same date, the electronic device 101 may include the acquired biometric information as the information of the corresponding period. For example, in case where the electronic device 101 checks a sixth time point (e.g., after the fifth time point 915, not shown) of measuring biometric information, the electronic device may measure user's blood sugar. For example, the electronic device 101 may determine, as the sixth time point, a time point at which the user starts a meal after the fifth time point 915. In case where the electronic device 101 determines the sixth time point, the electronic device 101 may again measure user's blood sugar after a designated time (e.g., thirty minutes) elapses. The electronic device 101 may check the kind of food that the user has based on a user's blood sugar variation, and may determine whether the user has a recommended menu.

And, the electronic device 101 may determine a user's health state (e.g., good, normal or bad) at a specific time point (e.g., 24:00) of one period based on biometric information checked during the one period. Here, in case where one day is determined as a period, a start (or reset) of a next period may be a time point at which the electronic device 101 checks 24:00, or may be a time point designated by a user.

The electronic device 101 may compare user's biometric information acquired during one period with biometric information of another period. Based on the comparison result, the electronic device 101 may check a variation of a user's health state, and may provide the checked result through the display 150. Based on the comparison result, the electronic device 101 may set or change a biometric time point, or may change a previously stored biometric time point as well.

The electronic device 101 describes, though not limited to, an example embodiment in which one period is one day, but may set various periods of one week, one month, and one year, and may generate statistics data according to a designated period based on acquired biometric information. The electronic device 101 may provide a variation of a user's health state based on the statistics data.

In the aforementioned description, each of the time points may be, though not limited to, time information stored in setting information of the electronic device 101, but may be also a time point after a designated time elapses from a time point at which the electronic device 101 is worn, or may be also a time point determined considering at least one another information (e.g., position information) together. Further, it is obvious that the aforementioned time points may be also time points determined based on a user's input.

FIG. 10 is a diagram illustrating an operation of determining a situation in which a user is placed based on acquired information in an electronic device according to various example embodiments.

According to various example embodiments, the electronic device 101 may determine a user situation to determine based on biometric information. To determine the user situation, the electronic device 101 may include, in a database, information (e.g., an emergency state level 1000) such as a data table and a data sheet which is divided into a plurality of steps correspondingly to the biometric information. The electronic device 101 may perform an operation that is set corresponding to the emergency state level 1000 corresponding to the biometric information. According to one example embodiment, in case of a step 4 (green), the electronic device 101 may determine a user's state as a normal state.

According to one example embodiment, in case where the electronic device 101 determines a user's abnormal situation step as a step 3 (yellow), the electronic device 101 may perform a designated operation that a user can be aware of. For example, the electronic device 101 may output a vibration using at least one motor. The electronic device 101 may determine that a user situation is a shock caused by low blood sugar, and may output a notification notifying a need for sugar intake to an output means (e.g., the display 150), or may output an audio (e.g., a voice notification) through a speaker.

According to one example embodiment, the electronic device 101 may determine that the user's abnormal situation step is a step 2 (orange). The electronic device 101 may send a call and/or transmit a designated message to a contact (e.g., a family) that is designated to an emergency calling number correspondingly to the step 2 (orange). In case where it is determined that the user situation is a fainting state, the electronic device 101 may perform an electric shock for correcting a user's posture or for awakening a user's consciousness, for a user.

According to one example embodiment, the electronic device 101 may determine that the user's abnormal situation step is a step 1 (red). The electronic device 101 may send a call and/or may transmit a designated message to a contact (e.g., 119 of South Korea or 911 of USA) that is designated to a state-designated emergency calling number correspondingly to the step 1 (red). In case where the electronic device 101 determines that a user's state is a situation requiring emergency relaxation activities, the electronic device 101 may perform a designated operation. For example, in case where the electronic device 101 determines that a user's fainting situation is a shock caused by low blood sugar and determines that it is an emergency (e.g., a blood sugar level is equal to or is less than a designated level) based on biometric information, the electronic device 101 may give insulin shot to the user.

According to one example embodiment, in case where the electronic device 101 determines that a user situation is a fainting situation and a non-breathing situation, the electronic device 101 may operate a defibrillator. For example, the electronic device 101 may be in a state of being wiredly or wirelessly coupled with the defibrillator located on the chest of the user. The electronic device 101 may real-time acquire biometric information correspondingly to the user situation, and may operate the defibrillator based on the biometric information.

As described above, the electronic device 101 may perform a designated operation based on a user situation that is determined based on biometric information.

FIG. 11 is a diagram illustrating an operation of performing a designated operation based on a user's position in an electronic device according to various example embodiments.

According to various example embodiments, the electronic device 101 may predict a user's designated operation based on time information and position information. Further, in case where a user is located in a non-designated position at a designated time, the electronic device 101 may determine this as an exceptional operation. According to one example embodiment, the electronic device 101 may include, in a list (e.g., a restaurant list), a position of a restaurant that the user often visits at a lunch time (e.g., from 11:30 to 12:30). For example, the electronic device 101 may include a list (e.g., a restaurant list) of position information (e.g., coordinates) that are set through a user input or position information (e.g., coordinates) that are set through at least once or more visits. In case where the user visits a specific restaurant 1110 (e.g., a Korean style restaurant 1110) included in the restaurant list at the lunch time, the electronic device 101 may store log data such as "You had a lunch in a Korean style restaurant 1110". When storing the log data, the electronic device 101 may store information such as a time at which the user arrives at the Korean style restaurant 1110, a staying time, and a leaving time.

According to various example embodiments, in case where the electronic device 101 detects that the user moves to a positon (e.g., a position 1120) not stored in the restaurant list at the lunch time, the electronic device 101 may output a notification of getting out of a movement expected area. When outputting the notification of getting out of the movement expected area, the electronic device 101 may output this notification at a time point of checking that it is a state in which a user movement is stopped for a designated time or more (e.g., ten to fifteen minutes or more). The electronic device 101 may determine, as a lunch place, a place (e.g., the position 1120) that the user is located at the lunch time, and may determine whether to include, in the restaurant list, position information (e.g., position information such as a coordinate) of the corresponding place (e.g., the position 1120). When including the corresponding place (e.g., the position 1120) in the restaurant list, the electronic device 101 may set a name of the corresponding place based on a user input.

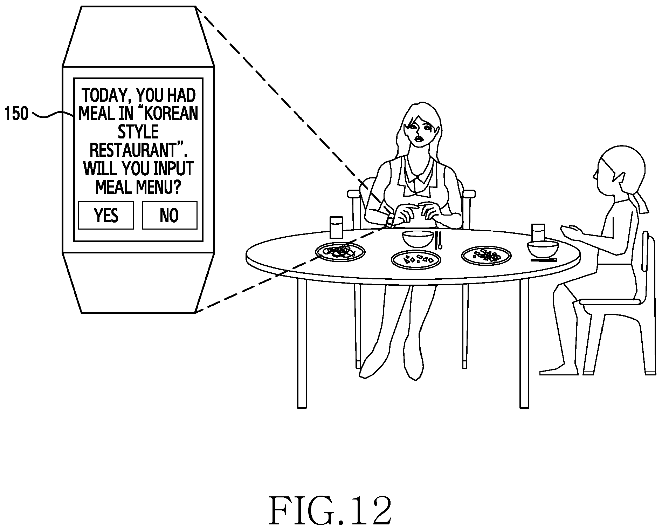

FIG. 12 is a diagram illustrating an operation dependent on user's biometric information variation in an electronic device according to various example embodiments.

According to various example embodiments, the electronic device 101 may check user's biometric information (e.g., periodically and/or continuously acquire the biometric information) in a specific condition and, in case where the electronic device 101 detects a change of a designated condition, the electronic device 101 may perform an operation corresponding to the change of the designated condition. According to one example embodiment, the electronic device 101 may check user's blood sugar by periods. In case where the electronic device 101 detects that user's blood sugar is varied within a designated time (e.g., thirty minutes) on a basis of a lunch time (e.g., 11:30 to 12:30), the electronic device 101 may determine that the user has a lunch. The electronic device 101 may determine a place where the user has a meal based on a place where the electronic device 101 is located at the lunch time. For example, in case where it is determined that the user is located in a "Korean style restaurant" or a building equipped with the "Korean style restaurant" at the lunch time, the electronic device 101 may determine that the user has a meal in the "Korean style restaurant". In other words, in case where it is determined that the user's blood sugar is varied within thirty minutes after the user is located in the "Korean style restaurant", the electronic device 101 may determine that the user has the meal in the "Korean style restaurant". The electronic device 101 may output a notification of having the meal in the "Korean style restaurant" to the user, and may provide a pop-up window of inquiring whether to input a meal menu. Based on the user input, the electronic device 101 may input the meal menu (if "Yes"), or may end without inputting the meal menu (if "No").

FIG. 13 is a diagram illustrating an operation of displaying a user situation that is determined based on biometric information in an electronic device according to various example embodiments.

According to various example embodiments, the electronic device 101 may store, in a database, an event repeated at a designated count or more based on device situation information and/or user's biometric information. For example, the electronic device 101 may check a rise of a blood pressure in a state of staying in a specific position as mentioned in operation 507 of FIG. 5. The electronic device 101 may determine, as a smoking situation, information that a user's blood pressure rises in a specific position with reference to the database. In case where the electronic device 101 repeatedly checks a rise of a blood pressure at a designated count (e.g., five times) or more in the aforementioned specific position, the electronic device 101 may generate, as a smoking event, the specific position and the blood pressure rise in the database. In case where the electronic device 101 detects a user who moves to a position stored as a smoking place, the electronic device 101 may predict that a user's movement intention is for smoking.

As described above, the electronic device 101 may store, in a database, as an event, a user's behavior repeated at a designated count or more based on device situation information and/or biometric information. In case where the corresponding event occurs later, despite the fact that the corresponding event is not an event stored in schedule information, the electronic device 101 may store a variation of user's biometric information as valid information predicting a user situation.

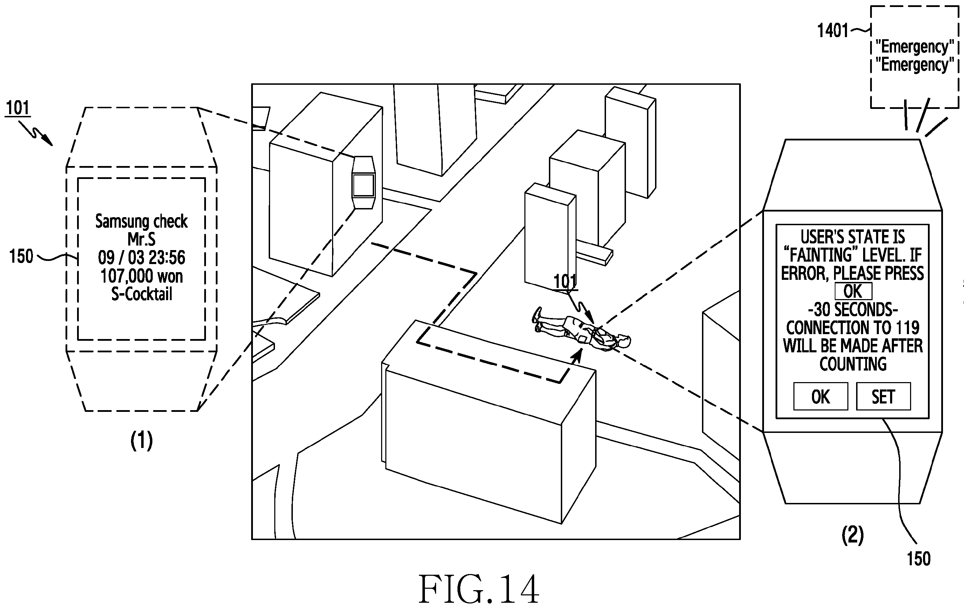

FIG. 14 is a diagram illustrating an operation dependent on user's biometric information variation in an electronic device according to various example embodiments.

According to various example embodiments, the electronic device 101 may detect a variation of user's biometric information after performing a specific operation. According to one example embodiment, the electronic device 101 may check user's payment information. For example, in case where a user pays with a credit card or a Point Of Sales or "POS" system, the electronic device 101 may receive a message about payment information (e.g., card payment information or POS payment information). The electronic device 101 may determine a user's behavior based on the received message. For example, the electronic device 101 may check that the user is consuming beverages based on a received message about payment information of (1) of FIG. 14. The electronic device 101 may check user's biometric information until the user returns home after drinking. When checking the user's biometric information, the electronic device 101 may check the user's biometric information even before a specific operation based on setting information, without being limited to checking the user's biometric information after the specific operation (e.g., receiving a message about payment information). When checking a variation of user's biometric information after the specific operation, the electronic device 101 may check a sudden biometric information variation, or biometric information defined as a user's abnormal state.

For example, the electronic device 101 may be in a state of periodically checking a heart rate of the user and/or a blood pressure. The electronic device 101 may detect a biometric information variation in which the heart rate of the user sharply falls or the blood pressure sharply falls (or rises). The electronic device 101 may determine a user's abnormal state such as a fainting state or a vomiting state based on the detected biometric information. The electronic device 101 may output, through the display 150 of the electronic device 101, a message (2) of checking whether the user is in the abnormal state. In case where the electronic device 101 cannot detect a user input during a designated time after the message outputting, the electronic device 101 may output a notification message 1401 of asking for help around. Further, the electronic device 101 may perform a designated operation (e.g., contacting an emergency call 119 and/or transmitting user's position information) for an emergency situation.

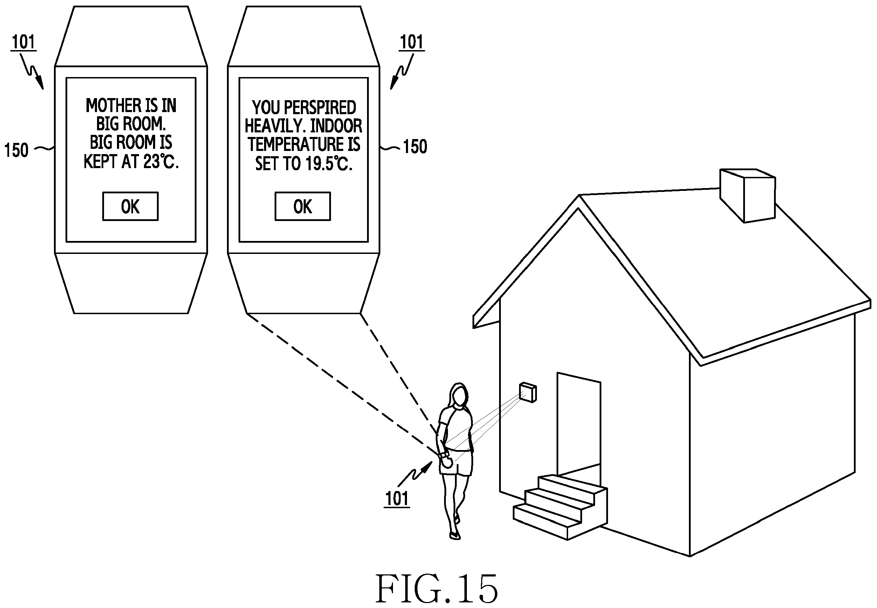

FIG. 15 is a diagram illustrating an operation of controlling another electronic device based on user's biometric information in an electronic device according to various example embodiments.

According to various example embodiments, the electronic device 101 may check log data of the electronic device 101 and/or biometric information of a user at a specific time point. According to one example embodiment, the electronic device 101 may check that the user arrives in front of a user's home after going out. For example, the electronic device 101 may check information about going out based on the log data, and may check the user who again arrives in front of the home thereafter. As a method of checking that the user goes out, the electronic device 101 may check, through a GPS, that the user becomes more distant from a position that is set as the user's home. Or, the electronic device 101 may check information about becoming more distant from the home based on a signal received from another electronic device (e.g., the second electronic device 102) located at a gate of the user's home. Likewise, according to one example embodiment checking that the user arrives at the home, the electronic device 101 may check, through the GPS, information about arriving at the home, or may determine the information based on a signal received from the another electronic device (e.g., the second electronic device 102) located at the gate of the user's home. In case where the electronic device 101 determines that the user arrives at the home, the electronic device 101 may acquire biometric information of the user.

According to one example embodiment, the electronic device 101 may acquire information about user's perspiration. In case where it is determined that the user perspires heavily, the electronic device 101 may decrease an indoor temperature of the user's home to a designated temperature (e.g., 19 degrees centigrade) and provide a pleasant environment to the user. According to one example embodiment, in case where it is determined that the user catches a cold based on perspiration information, user's body temperature and log data, the electronic device 101 may control the internal temperature of the user's home to a designated temperature (e.g., 23 degrees centigrade). When controlling the internal temperature of the user's home, the electronic device 101 may transmit a signal (e.g., a control signal) requesting to maintain the designated temperature to at least one another electronic device (e.g., an air conditioning device).

According to various example embodiments, when transmitting a control signal to at least one another electronic device (e.g., an air conditioning device), the electronic device 101 may determine this based on a user input and/or information received from at least one another electronic device (e.g., the electronic device 104). According to one example embodiment, the electronic device 101 may check information about mother's staying at a user's home, based on the user input. Or, the electronic device 101 may check the information by receiving mother's biometric information and/or mother's situation information from another electronic device (e.g., the electronic device 104) that a mother wears before a user goes out.

The electronic device 101 may check a mother's health state based on the mother's biometric information received from the electronic device 104. When the electronic device 101 controls an internal temperature of the user's home at a time point at which the user returns home as aforementioned, the electronic device 101 may separately control a temperature of a place where the mother is located based on the mother's health state. According to one example embodiment, in case where it is checked that the mother is located in a big room, when controlling the internal temperature of the user's home, the electronic device 101 may transmit a control signal to at least one another electronic device (e.g., an air conditioning device) to control a temperature of the remaining place excepting the big room.

According to various example embodiments, when the electronic device 101 acquires information of another electronic device (e.g., the electronic device 104), the electronic device 101 may receive the information through a designated network communication (e.g., a communication scheme such as Bluetooth communication, WiFi communication, infrared communication, and NFC), or may synchronize with the electronic device 104 through a designated server (e.g., the server 106). The aforementioned description has been made for that the electronic device 101 receives mother's biometric information and/or mother's situation information from the electronic device 104, but the information of the electronic device 104 may be received from the synchronized server 106 as well.

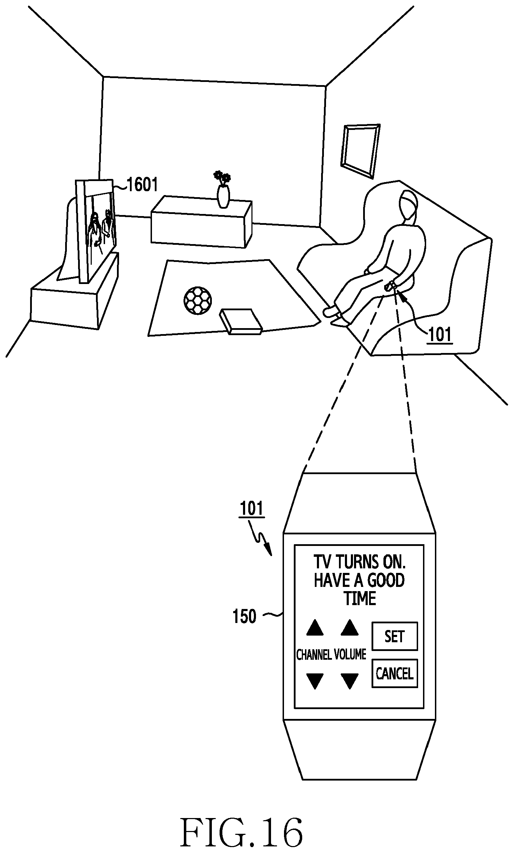

FIG. 16 is a diagram illustrating an operation of performing a designated operation based on user's biometric information at a specific time point in an electronic device according to various example embodiments.

According to various example embodiments, the electronic device 101 may perform a designated operation based on a position of a user at a specific time point. According to one example embodiment, in case where the user returns home at a specific time (e.g., after 8 p.m.), the electronic device 101 may include log data about watching on a TV 1601. The electronic device 101 may check information about that the user returns home after 08 p.m., and check that the user stays for a designated time (e.g., three seconds) or more at a sitting room (or a sitting room sofa) in which the TV 1601 is located, without movement. In case where the user stays for the designated time or more without movement, the electronic device 101 may control to power On the TV 1601. In case where the electronic device 101 controls to power On the TV 1601, the electronic device 101 may display a remote control interface of the TV 1601 on the display 150 of the electronic device 101 so that the user may use the electronic device 101 as a remote controller. Or, the electronic device 101 may perform an operation of the remote controller based on a user's voice input. Further, in case where it is determined that the user gets out of the TV 1601 (e.g., a designated distance or more from the TV 1601) for a designated time or more, the electronic device 101 may power off the TV 1601.

According to various example embodiments, the electronic device 101 may check, from log data, information about that a user prepares food at a specific time point (e.g., a time point designated as a mealtime) with staying at a home. In case where the electronic device 101 detects that the user moves to a kitchen at a specific time point, the electronic device 101 may control to open a relief valve of a gas range and/or may control to power on a ventilation fan. Further, in case where it is checked that the user gets out of the kitchen, the electronic device 101 may control to light off the kitchen and close the relief valve of the gas range, and may control to power off the ventilation fan after a designated time elapses.

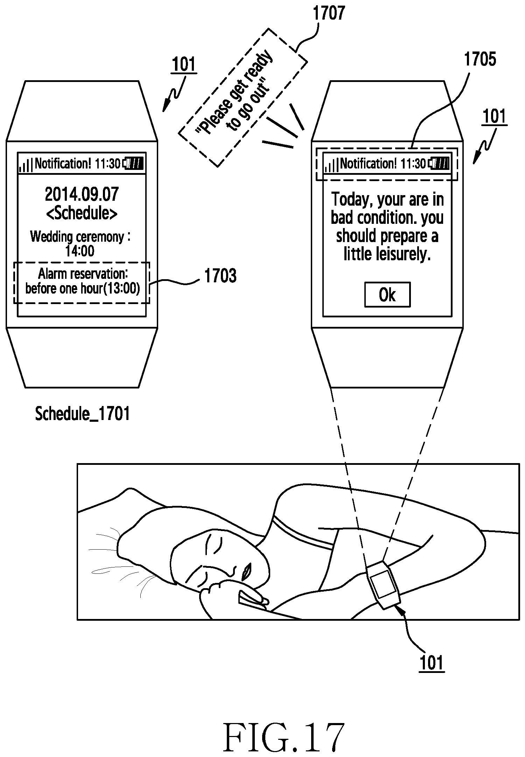

FIG. 17 is a diagram illustrating an operation of changing a designated condition based on user's biometric information in an electronic device according to various example embodiments.

According to various example embodiments, the electronic device 101 may check that a schedule for a wedding ceremony is set to 14:00, September 07, 2014 based on schedule information 1701, and may check that an alarm 1703 is set one hour before the time (e.g., 14:00) designated to the corresponding schedule. The electronic device 101 may check that a user's condition is an abnormal state based on user's biometric information. According to one example embodiment, the electronic device 101 may determine that the user catches a cold based on perspiration information of the user and/or body temperature information of the user. The electronic device 101 may predict that it will take a longer time than expected in user's performing a designated schedule in a state of a bad condition, or predict that a user will feel the stress of a daily movement. The electronic device 101 may control the designated alarm time 13:00 based on a user's condition and notify an alarm to the user. According to one example embodiment, in case where it is determined that the user catches a cold based on user's biomedical information, the electronic device 101 may output an alarm sound 1707 earlier a designated range time (e.g., thirty minutes) than the alarm time 13:00 designated to the schedule (e.g., the wedding ceremony). When the electronic device 101 outputs the alarm based on changed time information, the electronic device 101 may output (e.g., output through a speaker), to the user, a notification message 1705 that the user needs to prepare beforehand because he/she is in bad condition, together.

FIG. 18 is a diagram illustrating an operation of performing an operation of a designated schedule based on log data in an electronic device according to various example embodiments.

According to various example embodiments, when the electronic device 101 detects a user's movement, the electronic device 101 may check information about a transportation means such as whether the user uses a public transportation, whether the user uses a vehicle and/or whether the user uses a foot, based on various sensors included in the electronic device 101. Further, the electronic device 101 may also check information such as a movement speed of each transportation means or an average movement speed thereof as well. According to one example embodiment, the electronic device 101 may determine that the user uses the public transportation (e.g., a bus or subway) based on a route on which the user moves, a movement speed and/or traffic situation information, through an acceleration sensor and/or a GPS.

According to another example embodiment, the electronic device 101 may determine that the user uses public transportation (e.g., the bus or subway) based on car interval time information of a specific public transportation or movement route information thereof and a movement speed of a user. The electronic device 101 may check a schedule stored in schedule information based on the public transportation used for user's movement with reference to log data. In case where the electronic device 101 checks a position (e.g., S-place) stored in the schedule of the schedule information, the electronic device 101 may determine a transportation means that the user uses today in the log data, and may determine information of a route to the stored position (e.g., S-place) by the corresponding transportation means.

The electronic device 101 may provide the route information to the user, in addition to information such as an expected arrival time and an expected starting time. According to one example embodiment, the electronic device 101 may provide the user with a menu 1801 having options selectable to alter the transportation means corresponding to the route information. In case where the transportation means is altered, the electronic device 101 may provide the user with information such as route information, an expected arrival time, and an expected starting time corresponding to the altered transportation means.

According to one example embodiment, the electronic device 101 may provide a customized function based on device situation information and user's biometric information correspondingly to an event occurring in an unexpected situation, thereby effectively providing a user with a function necessary for a specific situation.

According to one example embodiment, the processor 120 may acquire biometric information of a user in a state in which the electronic device 101 is attached to or worn on a part of the body of the user. According to one example embodiment, in case where it is repeated at a designated count or more that biometric information acquired in a specific situation satisfies a designated condition, the processor 120 may store an event corresponding to the specific situation and the biometric information.

According to one example embodiment, the processor 120 may perform an operation corresponding to a designated condition. According to one example embodiment, the processor 120 may perform the operation corresponding to the designated condition based on a specific situation.

According to one example embodiment, the processor 120 may detect a user's blood sugar variation based on acquired biometric information. According to one example embodiment, the processor 120 may measure user's blood sugar after a designated time elapses from a time point of detecting the user's blood sugar variation. Each of the aforementioned constituent elements of the electronic device according to various example embodiments of the present disclosure may include one or more components, and a name of the corresponding constituent element may vary according to the type of the electronic device. The electronic device according to various example embodiments of the present disclosure may include at least one of the aforementioned constituent elements, and may omit some constituent elements or may further include additional other constituent elements. Also, some of the constituent elements of the electronic device according to various example embodiments of the present disclosure may be combined and implemented as one entity, thereby identically performing functions of the corresponding constituent elements before combination.

The above-described embodiments of the present disclosure can be implemented in hardware, firmware or via the execution of software or computer code that can be stored in a recording medium such as a compact disc ROM (CD-ROM), a DVD, a magnetic tape, a RAM, a floppy disk, a hard disk, or a magneto-optical disk or computer code downloaded over a network originally stored on a remote recording medium or a non-transitory machine readable medium and to be stored on a local recording medium, so that the methods described herein can be rendered via such software that is stored on the recording medium using a general purpose computer, or a special processor or in programmable or dedicated hardware, such as an application specific integrated circuit (ASIC) or field programmable gate array (FPGA). As would be understood in the art, the computer, the processor, microprocessor processor or the programmable hardware include memory components, e.g., RAM, ROM, Flash, etc. that may store or receive software or computer code that when accessed and executed by the computer, processor or hardware implement the processing methods described herein.

Further, the 'module' may be provided as a computer-readable storage media storing one or more programs (or programming modules and applications). For instance, the software may be implemented by an instruction stored in a computer-readable storage media in a form of the programming module. The one or more programs may include instructions for enabling an electronic device to execute methods according to an embodiment stated in the claims and/or specification of the present disclosure. If the instruction is executed by one or more processors (e.g., the processor 120), the one or more processors may perform a function corresponding to the instruction. The computer-readable storage media may be, for example, the memory 130. At least a part of the programming module may be, for example, implemented (e.g., executed) by the processor 120. At least a part of the programming module may include, for example, a module, a program, a routine, sets of instructions, or a process, etc. for performing one or more functions.

According to various example embodiments, an electronic device may include a computer-readable storage media storing a program for performing the operations of acquiring device situation information of the electronic device, checking a specific situation based on the device situation information, in case where the specific situation is not a designated event, acquiring user's biometric information, and in case where the acquired biometric information satisfies a designated condition, storing the specific situation and the biometric information.

According to one example embodiment, the electronic device 101 may include a computer-readable storage media storing a program for performing the operations of checking a specific time point and user situation information and performing a function corresponding to the specific time point and the user situation information based on log data.

According to one example embodiment, the electronic device 101 may include a computer-readable storage media storing a program for performing the operations of acquiring device situation information of the electronic device 101, checking a specific situation based on the device situation information, in case where the specific situation is not a designated event, acquiring user's biometric information, and storing information of the specific situation and the biometric information.

According to one example embodiment, the electronic device 101 may include a computer-readable storage media storing a program for performing the operations of determining a user's mealtime based on device situation information of the electronic device 101, acquiring user's biometric information at a designated time interval, determining the ending of the mealtime based on the biometric information, determining a medication time point base on the ending of the mealtime, and outputting a notification of the medication time point.

Further, the program may be stored in an attachable storage device capable of accessing the electronic device through a communication network such as the Internet, an Intranet, a Local Area Network (LAN), a Wide LAN (WLAN), or a Storage Area Network (SAN), or a communication network implemented in combination of them. This storage device may connect to the electronic device through an external port. Also, a separate storage device on the communication network may connect to a portable electronic device as well. The aforementioned hardware device may be configured to be activated as one or more software modules so as to perform operations of various example embodiments of the present disclosure, and vice versa.

The module or programming module according to various embodiments of the present disclosure may include at least one or more of the aforementioned constituent elements, or omit some of the aforementioned constituent elements, or further include additional other constituent elements. Operations carried out by the module, the programming module or other constituent elements according to various embodiments of the present disclosure may be executed in a sequential, parallel, repeated or heuristic method. Also, some operations may be executed in different order or may be omitted, or other operations may be added.

While the present disclosure has been shown and described with reference to various embodiments thereof, it will be understood by those skilled in the art that various changes in form and details may be made therein.

The above-described embodiments of the present disclosure can be implemented in hardware, firmware or via the execution of software or computer code that can be stored in a recording medium such as a CD ROM, a Digital Versatile Disc (DVD), a magnetic tape, a RAM, a floppy disk, a hard disk, or a magneto-optical disk or computer code downloaded over a network originally stored on a remote recording medium or a non-transitory machine readable medium and to be stored on a local recording medium, so that the methods described herein can be rendered via such software that is stored on the recording medium using a general purpose computer, or a special processor or in programmable or dedicated hardware, such as an ASIC or FPGA. As would be understood in the art, the computer, the processor, microprocessor controller or the programmable hardware include memory components, e.g., RAM, ROM, Flash, etc. that may store or receive software or computer code that when accessed and executed by the computer, processor or hardware implement the processing methods described herein. In addition, it would be recognized that when a general purpose computer accesses code for implementing the processing shown herein, the execution of the code transforms the general purpose computer into a special purpose computer for executing the processing shown herein. Any of the functions and steps provided in the Figures may be implemented in hardware, software or a combination of both and may be performed in whole or in part within the programmed instructions of a computer.

## Claims

1. A method operable in a wearable device (101), the method comprising:
determining an acquisition condition for acquiring biometric information of a user of the wearable device, based on movement information acquired by a movement detection unit (170), wherein determining the acquisition condition for acquiring biometric information of the user comprises:
determining whether a state of the wearable device (101) is stationary for at least a previously designated time period based on acquired movement information;
if the state of the wearable device (101) is stationary for at least the designated time period, acquiring location information of the user;
determining whether the acquired location of the user corresponds with a designated situation, wherein the designated situation is a situation corresponding to schedule information of the user stored in the memory (130), wherein the schedule information comprises position and time information of the user; and
if the acquired location does not correspond with a designated situation, acquiring the biometric information of the user;
storing, in at least one of the memory (130) and an external server (106), the determined acquisition condition and the biometric information of the user;
comparing the stored biometric information of the user with a predesignated setting value; and
controlling the wearable device (101) or another electronic device (102, 104) communicatively coupled with the wearable device (101) in accordance with the comparison result.

2. The method of claim 1, further comprising:
the location information indicates at least one of a latitude, a longitude, a height, a distance from another electronic device, and whether the wearable device is indoors; and
acquiring the biometric information if the wearable device (101) moves out of a set geographic range at a designated time.

3. The method of claim 1, wherein controlling the wearable device (101) or the another electronic device (102, 104) communicatively coupled with the wearable device (101) in accordance with the comparison result further comprises transmitting determined health state information to a predesignated electronic device of a medical institution, or transmitting an emergency signal to the another electronic device (102, 104).

4. The method of claim 1, wherein controlling the wearable device (101) or the another electronic device (102, 104) communicatively coupled with the wearable device (101) in accordance with the comparison result further comprises at least one of:
changing an operation mode of the wearable device (101),
outputting a notification message in the wearable device (101), and
transmitting the notification message to the another electronic device (102, 104) coupled with the wearable device (101).

5. A wearable device (101) comprising:
a movement detection unit (170),
a position detection unit (170),
a biometric information detection unit (170);
a wireless communication unit (160);
a memory (130); and
a processor (120) configured to:
determine an acquisition condition for acquiring biometric information of a user of the wearable device based on movement information acquired by the movement detection unit (170), wherein determining the acquisition condition for acquiring biometric information of the user comprises:
determining whether a state of the wearable device (101) is stationary for at least a previously designated time period based on acquired movement information;
if the state of the wearable device (101) is stationary for at least the designated time period, acquiring location information of the user;
determining whether the acquired location of the user corresponds with a designated situation, wherein the designated situation is a situation corresponding to schedule information of the user stored in the memory (130) wherein the schedule information comprises position and time information of the user; and
if the acquired location does not correspond with a
designated situation, acquiring the biometric information of the user,
store, in at least one of the memory (130) and an external server (106), the determined acquisition condition and the biometric information of the user,
compare the stored biometric information of the user with a predesignated setting value, and
control the wearable device (101) or another electronic device (102, 104) communicatively coupled with the wearable device (101) in accordance with the comparison result.

6. The device of claim 5, wherein the biometric information detection unit (170) comprises an optical sensor including a light receiving unit and a light emitting unit, and an electrical sensor attached to skin or clothing and is configured to detect an electrical signal.

7. The device of claim 5, wherein the location information indicates at least one of a latitude, a longitude, a height, a distance from the another electronic device, and whether the wearable device is indoors, the processor further configured to: acquire the biometric information if the wearable device (101) moves out of a set geographic range at a designated time.

8. The device of claim 5, wherein the processor (120) is configured to provide health state information to a predesignated electronic device of a medical institution through the wireless communication unit (160), or transmit an emergency signal to the another electronic device (102, 104),
wherein the health state information includes at least one of among "good," "bad," "emergency," and "serious."

9. The device of claim 5, wherein controlling the wearable device (101) or another electronic device (102, 104) in accordance with the comparison result further comprises at least one of:
changing an operation mode of the wearable device (101), and
output a notification message in the wearable device (101).

## Patentansprüche

1. Verfahren, das in einer tragbaren Vorrichtung (101) betreibbar ist, wobei das Verfahren Folgendes umfasst:
Bestimmen einer Erfassungsbedingung zum Erfassen von biometrischen Informationen eines Benutzers der tragbaren Vorrichtung, basierend auf Bewegungsinformationen, die durch eine Bewegungsdetektionseinheit (170) erfasst werden, wobei das Bestimmen der Erfassungsbedingung zum Erfassen von biometrischen Informationen des Benutzers Folgendes umfasst:
Bestimmen, ob ein Zustand der tragbaren Vorrichtung (101) für zumindest eine zuvor bestimmte Zeitdauer stationär ist, basierend auf erfassten Bewegungsinformationen;
wenn der Zustand der tragbaren Vorrichtung (101) für zumindest die bestimmte Zeitdauer stationär ist, Erfassen von Standortinformationen des Benutzers;
Bestimmen, ob der erfasste Standort des Benutzers einer bestimmten Situation entspricht, wobei die bestimmte Situation eine Situation ist, die Zeitplaninformationen des Benutzers entspricht, die in dem Speicher (130) gespeichert sind, wobei die Zeitplaninformationen Positions- und Zeitinformationen des Benutzers umfassen; und
wenn der erfasste Standort nicht mit einer bestimmten Situation übereinstimmt, Erfassen der biometrischen Informationen des Benutzers;
Speichern, in zumindest einem von dem Speicher (130) und einem externen Server (106), der bestimmten Erfassungsbedingung und der biometrischen Informationen des Benutzers;
Vergleichen der gespeicherten biometrischen Informationen des Benutzers mit einem vorbestimmten Einstellwert; und
Steuern der tragbaren Vorrichtung (101) oder einer anderen elektronischen Vorrichtung (102, 104), die kommunikativ mit der tragbaren Vorrichtung (101) gekoppelt ist, gemäß dem Vergleichsergebnis.

2. Verfahren nach Anspruch 1, ferner umfassend:
die Standortinformationen geben zumindest eines von einem Breitengrad, einem Längengrad, einer Höhe, einer Entfernung von einer anderen elektronischen Vorrichtung und ob die tragbare Vorrichtung innen ist, an; und
Erfassen der biometrischen Informationen, wenn sich die tragbare Vorrichtung (101) zu einer bestimmten Zeit aus einem festgelegten geografischen Bereich herausbewegt.

3. Verfahren nach Anspruch 1, wobei das Steuern der tragbaren Vorrichtung (101) oder der anderen elektronischen Vorrichtung (102, 104), die kommunikativ mit der tragbaren Vorrichtung (101) gekoppelt ist, gemäß dem Vergleichsergebnis ferner das Übertragen von bestimmten Gesundheitszustandsinformationen an eine vorbestimmte elektronische Vorrichtung einer medizinischen Einrichtung oder das Übertragen eines Notfallsignals an die andere elektronische Vorrichtung (102, 104) umfasst.

4. Verfahren nach Anspruch 1, wobei das Steuern der tragbaren Vorrichtung (101) oder der anderen elektronischen Vorrichtung (102, 104), die kommunikativ mit der tragbaren Vorrichtung (101) gekoppelt ist, gemäß dem Vergleichsergebnis ferner zumindest eines des Folgenden umfasst:
Ändern eines Betriebsmodus der tragbaren Vorrichtung (101),
Ausgeben einer Benachrichtigungsnachricht in der tragbaren Vorrichtung (101), und
Übertragen der Benachrichtigungsnachricht an die andere elektronische Vorrichtung (102, 104), die mit der tragbaren Vorrichtung (101) gekoppelt ist.

5. Tragbare Vorrichtung (101), umfassend:
eine Bewegungsdetektionseinheit (170),
eine Positionsdetektionseinheit (170),
eine Detektionseinheit für biometrische Informationen (170);
eine drahtlose Kommunikationseinheit (160);
einen Speicher (130); und
einen Prozessor (120), der für Folgendes konfiguriert ist:
Bestimmen einer Erfassungsbedingung zum Erfassen von biometrischen Informationen eines Benutzers der tragbaren Vorrichtung basierend auf Bewegungsinformationen, die durch die Bewegungsdetektionseinheit (170) erfasst werden, wobei das Bestimmen der Erfassungsbedingung zum Erfassen von biometrischen Informationen des Benutzers Folgendes umfasst:
Bestimmen, ob ein Zustand der tragbaren Vorrichtung (101) für zumindest eine zuvor bestimmte Zeitdauer stationär ist, basierend auf erfassten Bewegungsinformationen;
wenn der Zustand der tragbaren Vorrichtung (101) für zumindest die bestimmte Zeitdauer stationär ist, Erfassen von Standortinformationen des Benutzers;
Bestimmen, ob der erfasste Standort des Benutzers einer bestimmten Situation entspricht, wobei die bestimmte Situation eine Situation ist, die Zeitplaninformationen des Benutzers entspricht, die in dem Speicher (130) gespeichert sind, wobei die Zeitplaninformationen Positions- und Zeitinformationen des Benutzers umfassen; und
wenn der erfasste Standort nicht einer bestimmten Situation entspricht, Erfassen der biometrischen Informationen des Benutzers,
Speichern, in zumindest einem von dem Speicher (130) und einem externen Server (106), der bestimmten Erfassungsbedingung und der biometrischen Informationen des Benutzers,
Vergleichen der gespeicherten biometrischen Informationen des Benutzers mit einem vorbestimmten Einstellwert, und
Steuern der tragbaren Vorrichtung (101) oder einer anderen elektronischen Vorrichtung (102, 104), die kommunikativ mit der tragbaren Vorrichtung (101) gekoppelt ist, gemäß dem Vergleichsergebnis.

6. Vorrichtung nach Anspruch 5, wobei die Detektionseinheit für biometrische Informationen (170) einen optischen Sensor, der eine Lichtempfangseinheit und eine Lichtemissionseinheit beinhaltet, und einen elektrischen Sensor umfasst, der an Haut oder Kleidung angebracht ist und konfiguriert ist, um ein elektrisches Signal zu detektieren.

7. Vorrichtung nach Anspruch 5, wobei die Standortinformationen zumindest eines von einem Breitengrad, einem Längengrad, einer Höhe, einer Entfernung von der anderen elektronischen Vorrichtung und ob die tragbare Vorrichtung innen ist, angeben, wobei der Prozessor ferner für Folgendes konfiguriert ist: Erfassen der biometrischen Informationen, wenn sich die tragbare Vorrichtung (101) zu einer bestimmten Zeit aus einem festgelegten geografischen Bereich herausbewegt.

8. Vorrichtung nach Anspruch 5, wobei der Prozessor (120) konfiguriert ist, um Gesundheitszustandsinformationen an eine vorbestimmte elektronische Vorrichtung einer medizinischen Einrichtung durch die drahtlose Kommunikationseinheit (160) bereitzustellen oder ein Notfallsignal an die andere elektronische Vorrichtung (102, 104) zu übertragen,
wobei die Gesundheitszustandsinformationen zumindest eines von "gut", "schlecht", "Notfall" und "ernst" beinhalten.

9. Vorrichtung nach Anspruch 5, wobei das Steuern der tragbaren Vorrichtung (101) oder einer anderen elektronischen Vorrichtung (102, 104) gemäß dem Vergleichsergebnis ferner zumindest eines des Folgenden umfasst:
Ändern eines Betriebsmodus der tragbaren Vorrichtung (101), und
Ausgeben einer Benachrichtigungsnachricht in der tragbaren Vorrichtung (101).

## Revendications

1. Procédé utilisable dans un dispositif portable (101), le procédé comprenant :
la détermination d'une condition d'acquisition pour acquérir des informations biométriques d'un utilisateur du dispositif portable, sur la base d'informations de mouvement acquises par une unité de détection de mouvement (170), dans lequel la détermination de la condition d'acquisition pour acquérir des informations biométriques de l'utilisateur comprend :
la détermination si un état du dispositif portable (101) est stationnaire pendant au moins une période de temps préalablement désignée sur la base d'informations de mouvement acquises ;
si l'état du dispositif portable (101) est stationnaire pendant au moins la période de temps désignée, l'acquisition d'informations de localisation de l'utilisateur ;
la détermination si la localisation acquise de l'utilisateur correspond à une situation désignée, dans lequel la situation désignée est une situation correspondant à des informations de calendrier de l'utilisateur stockées dans la mémoire (130), dans lequel les informations de calendrier comprennent des informations de position et d'heure de l'utilisateur ; et
si la localisation acquise ne correspond pas à une situation désignée, l'acquisition des informations biométriques de l'utilisateur ;
le stockage, dans au moins l'un de la mémoire (130) et d'un serveur externe (106), de la condition d'acquisition déterminée et des informations biométriques de l'utilisateur ;
la comparaison des informations biométriques stockées de l'utilisateur à une valeur de réglage prédésignée ; et
la commande du dispositif portable (101) ou d'un autre dispositif électronique (102, 104) couplé en communication avec le dispositif portable (101) conformément au résultat de la comparaison.

2. Procédé selon la revendication 1, comprenant en outre :
les informations de localisation indiquent au moins l'un parmi une latitude, une longitude, une hauteur, une distance par rapport à un autre dispositif électronique, et si le dispositif portable se trouve en intérieur ; et
l'acquisition des informations biométriques si le dispositif portable (101) se déplace en dehors d'une plage géographique définie à un moment désigné.

3. Procédé selon la revendication 1, dans lequel la commande du dispositif portable (101) ou de l'autre dispositif électronique (102, 104) couplé en communication avec le dispositif portable (101) conformément au résultat de la comparaison comprend en outre la transmission d'informations d'état de santé déterminées à un dispositif électronique prédésigné d'un établissement médical, ou la transmission d'un signal d'urgence à l'autre dispositif électronique (102, 104).

4. Procédé selon la revendication 1, dans lequel la commande du dispositif portable (101) ou de l'autre dispositif électronique (102, 104) couplé en communication avec le dispositif portable (101) conformément au résultat de la comparaison comprend en outre au moins l'un parmi :
le changement d'un mode de fonctionnement du dispositif portable (101),
l'émission d'un message de notification dans le dispositif portable (101), et
la transmission du message de notification à l'autre dispositif électronique (102, 104) couplé au dispositif portable (101).

5. Dispositif portable (101) comprenant :
une unité de détection de mouvement (170),
une unité de détection de position (170),
une unité de détection d'informations biométriques (170) ;
une unité de communication sans fil (160) ;
une mémoire (130) ; et
un processeur (120) configuré pour :
déterminer une condition d'acquisition pour acquérir des informations biométriques d'un utilisateur du dispositif portable sur la base d'informations de mouvement acquises par l'unité de détection de mouvement (170), dans lequel la détermination de la condition d'acquisition pour acquérir des informations biométriques de l'utilisateur comprend :
la détermination si un état du dispositif portable (101) est stationnaire pendant au moins une période de temps préalablement désignée sur la base d'informations de mouvement acquises ;
si l'état du dispositif portable (101) est stationnaire pendant au moins la période de temps désignée, l'acquisition d'informations de localisation de l'utilisateur ;
la détermination si la localisation acquise de l'utilisateur correspond à une situation désignée, dans lequel la situation désignée est une situation correspondant à des informations de calendrier de l'utilisateur stockées dans la mémoire (130) dans lequel les informations de calendrier comprennent des informations de position et d'heure de l'utilisateur ; et
si la localisation acquise ne correspond pas à une situation désignée, l'acquisition des informations biométriques de l'utilisateur,
stocker, dans au moins l'un de la mémoire (130) et d'un serveur externe (106), la condition d'acquisition déterminée et des informations biométriques de l'utilisateur,
comparer les informations biométriques stockées de l'utilisateur à une valeur de réglage prédésignée, et
commander le dispositif portable (101) ou un autre dispositif électronique (102, 104) couplé en communication avec le dispositif portable (101) conformément au résultat de la comparaison.

6. Dispositif selon la revendication 5, dans lequel l'unité de détection d'informations biométriques (170) comprend un capteur optique comprenant une unité de réception de lumière et une unité d'émission de lumière, et un capteur électrique attaché à la peau ou aux vêtements et est configuré pour détecter un signal électrique.

7. Dispositif selon la revendication 5, dans lequel les informations de localisation indiquent au moins l'un parmi une latitude, une longitude, une hauteur, une distance par rapport à l'autre dispositif électronique, et si le dispositif portable se trouve en intérieur, le processeur étant en outre configuré pour : acquérir les informations biométriques si le dispositif portable (101) se déplace en dehors d'une plage géographique définie à un moment désigné.

8. Dispositif selon la revendication 5, dans lequel le processeur (120) est configuré pour fournir des informations d'état de santé à un dispositif électronique prédésigné d'un établissement médical via l'unité de communication sans fil (160), ou transmettre un signal d'urgence à l'autre dispositif électronique (102, 104),
dans lequel les informations d'état de santé comprennent au moins l'un parmi « bon », « mauvais », « urgence » et « grave ».

9. Dispositif selon la revendication 5, dans lequel la commande du dispositif portable (101) ou d'un autre dispositif électronique (102, 104) conformément au résultat de la comparaison comprend en outre au moins l'un parmi :
le changement d'un mode de fonctionnement du dispositif portable (101), et
l'émission d'un message de notification dans le dispositif portable (101).
